Europäisches Patentamt

European Patent Office

Office européen des brevets

(11) Veröffentlichungsnummer: **0 079 617**
**B1**

(12) EUROPÄISCHE PATENTSCHRIFT

(45) Veröffentlichungstag der Patentschrift: 30.09.87

(21) Anmeldenummer: 82110594.7

(22) Anmeldetag: 16.11.82

(51) Int. Cl.⁴: **A 61 B 5/02**

(54) Ablassventil für ein Blutdruckmessgerät.

(30) Priorität: 17.11.81 DE 3145658
26.08.82 DE 3231839

(43) Veröffentlichungstag der Anmeldung:
25.05.83 Patentblatt 83/21

(45) Bekanntmachung des Hinweises auf die
Patenterteilung:
30.09.87 Patentblatt 87/40

(84) Benannte Vertragsstaaten:
AT CH DE FR GB IT LI NL SE

(56) Entgegenhaltungen:
EP-A-0 014 977
DE-A-2 001 348
DE-C-3 117 546
US-A-3 030 945
US-A-3 489 172
US-A-3 875 961

(73) Patentinhaber: Firma Richard Kallmeyer
Im Farchet 15 Postfach 1320
D-8170 Bad Tölz (DE)

(72) Erfinder: Stiebler, Franz
Am Ried 8
D-8170 Bad Tölz (DE)

(74) Vertreter: Schwabe, Hans-Georg, Dipl.-Ing. et al
Patentanwälte Schwabe, Sandmair, Marx
Stuntzstrasse 16
D-8000 München 80 (DE)

Courier Press, Leamington Spa, England.

**Beschreibung**

Die Erfindung betrifft ein Ablaßventil mit Schnellablaßfunktion, für ein Blutdruckmeßgerät mit einer Pumpe zum Aufpumpen einer Manschette, das aus einer ersten, stabilen Stellung in eine stabile Schnellablaßstellung überführbar ist.

Es sind schon Schnellablaßventile bekannt (s. z.B. DE—GM—1 995 092 oder DE—OS—20 01 348), bei denen der Weg für das abzulassende Fluid beispielsweise durch den gegen die Kraft einer Feder gerichteten Druck der Hand freigegeben wird. Durch die Feder wird zwar beim Loslassen eine Rückstellung erzielt, jedoch ist es nachteilig, daß die Vorrichtung während des ganzen Schnellablaßvorganges aktiv von der Untersuchungsperson betätigt werden muß, so daß diese sich nicht schon anderweitig, beispielsweise mit dem Abnehmen der Manschette oder dem Notieren der gemessenen Werte, beschäftigen kann.

Z.B. aus der gattungsbildenden DE—OS—27 58 895 ist er darüber hinaus bekannt, eine Rastung vorzusehen, so daß das Ablaßventil in der Schnellablaßstellung verbleiben kann.

Dies hat jedoch den Nachteil, daß häufig die Rückstellung vergessen wird und erst nach vergeblichen Pumpversuchen das Ventil weider geschlossen wird.

Die Erfindung schafft daher ein Ablaßventil mit Schnellablaßfunktion der angegebenen, aus der DE—OS—27 58 895 bekannten Gattung, das so weitergebildet ist, daß das mit dem Schnellablaß versehene Gerät nach dem Schnellablaß ohne erneute Handhahung des Schnellablaßventils jederzeit wieder betriebsbereit ist.

Dies wird erfindungsgemäß dadurch erreicht, daß das Ablaßventil durch die Aufpumpenbetätigung der Pumpe in die erste, stabile Stellung übergeführt wird. Dies kann grundsätzlich auf verschiedene Weise geschehen. Beispielsweise kann auch eine Rastung für das Ablaßventil, das durch die Rastung in seiner Schnellablaßstellung verbleibt, durch die Pumpenbetätigung freigegeben werden, beispielsweise dadurch, daß das Rastglied mit der elastisch verformbaren Wandung eines handbetätigbaren Gebläseballes verbunden ist.

Besonders bevorzugt wird jedoch das Ablaßventil durch den Druck oder Sog der Pumpe in die erste Stellung übergeführt. Dies geschieht bevorzugt durch Druck, da der Sog der Pumpe beispielsweise bei einem Gebläseball durch dessen Aufbau vorgegeben ist, während sich der Druck beispielsweise bei einem handbetätigten Gebläseball noch steigern läßt, so daß bei der Überführung durch Fertigungstoleranzen etwa benötigte unterschiedliche Kräfte leicht aufgebracht werden können.

Besonders bevorzugt weist das Ablaßventil ein Ventilgehäuse mit im wesentlichen zylindrischen Innenraum auf, an den die Verbindung zur Pumpe, die Verbindung zur Manschette und die Atmosphäre angeschlossen sind, und im zylindrischen Innenraum ist ein entsprechender, im wesentlichen zylindrischer Steuerkörper verstellbar angeordnet, der in einer ersten Stellung die Verbindung zwischen Pumpenanschluß und Manschettenanschluß unter Abdichtung gegen die Atmosphäre und in einer zweiten Stellung (Schnellablaßstellung) die Verbindung zwischen dem Manschettenanschluß und der Atmosphäre unter Abdichtung gegen den Pumpenanschluß derart, daß ein Pumpendruck (oder gegebenenfalls -sog) den zylindrischen Steuerkörver in die erste Stellung verstellt, herstellt oder erlaubt. Dies hat den Vorteil einer besonders einfachen Herstellbarkeit eines entsprechenden Ablaßventils mit Schnellablaßfunktion. Grundsätzlich kann dabei die jeweilige Verbindung zwischen den Anschlüssen durch Kanäle im zylindrischen Steuerkörper hergestellt werden, wobei die Kanalöffnungen in den unterschiedlichen Lagen jeweils an die geeigneten Anschlußöffnungen angeschlossen sind. Dabei kann die Verstellung bei einem im wesentlichen kreiszylindrischen Steuerkörper in einem im wesentlichen kreiszylindrischen Innenraum auch durch Drehung des Steuerkörpers um seine Zylinderachse erfolgen. Der Pumpemdruck wirkt gegen geeignete Flächen des zylindrischen Körpers derart, daß dieser sich in seine erste Stellung verdreht oder verschiebt.

Besonders bevorzugt sind die Durchlaßöffnung zur Pumpe und die Durchlaßöffnung zur Manschette in Axialrichtung des Innenraums beabstandet angeordnet, ist der Steuerkörper im Innenraum des Ventilgehäuses in dessen Axialrichtung verschiebbar und unterteilt den Innenraum in zwei voneinander abgedichtete Teilräume variabler Größe, ist der Steuerkörper in zwei Lagen verschiebbar, in deren erster beide Durchlaßöffnungen in den ersten im übrigen abgeschlossenen Teilraum münden und mündet in der zweiten Lage die Durchlaßöffnung zur Pumpe in den ersten Teilraum und mündet die Durchlaßöffnung zur Manschette in den zweiten Teilraum, der wenigstens in der zweiten Verschiebelage des Steuerkörpers zur Atmosphäre hin offen ist.

Dadurch wird insbesondere ein besonders einfacher und zweckmäßiger Aufbau des Ablaßventils mit Schnellablaßfunktion erzielt. Die Durchlaßöffnungen sind jene Öffnungen, die in der Mantelwand des zylindrischen Innenraumes, aber auch in den Abschlußwänden dieses Innenraumes ausgebildet sein können. Dadurch, daß die Durchlaßöffnung zur Pumpe in den in der Schnellablaßstellung im übrigen dichtend abgeschlossenen ersten Teilraum mündet, kann sich durch Pumpenbetätigung in diesem ersten Teilraum ein Druck aufbauen, der den zylindrischen Steuerkörper so verschiebt, daß er auch die Durchlaßöffnung, die den Innenraum mit der Manschette verbindet, mit dem ersten Teilraum verbindet, der im übrigen gegen die Atmosphäre abgedichtet ist, so daß der Pumpvorgang nach bzw. schon mit dem ersten Pumpenstoß in üblicher Weise bei geschlossenem Schnellablaß durchgeführt werden kann. Durch axiales Verschieben des Steuerkörpers in die Schnellablaßlage wird der abgeschlossene Teilraum weiderum gegen die Durchlaßöffnung zur Manschette abgedichtet, und mündet die Durchlaßöffnung zur Manschette in den zweiten Teilraum, der in dieser Lage des Steuerkörpers zur Atmosphäre hin offen ist. Dieser zweite Teilraum kann im übrigen stets zur Atmosphäre hin offen sein, soweit der Steuerkörper seine Abdichtung gegen den ersten Teilraum sicherstellt.

Besonders bevorzugt sind der Anschluß der Pumpe, der Anschluß an die Manschette und der Anschluß an die Atmosphäre in dieser Reihenfolge in Axialrichtung gesehen an den Innenraum des Ventilgehäuses angeschlossen und ist der zylindrische Steuerkörper zumindest über einen Teil seiner Länge, der jedoch nicht größer ist als der Abstand zwischen den einander zugewandten Ründern der Anschlüsse, dichtend und gleitbar mit der Innenwand des Gehäuses mit zylindrischem Innernraum verbunden. Dabei ist der dichtende Bereich in der ersten Stellung zwischen dem Anschluß zur Manschette und zur Atmosphäre und in der Schnellablaßstellung zwischen dem Anschluß zur Manschette und dem Druckanschluß zur Pumpe angeordnet. Somit kann der Anschluß (d.h. die Durchlaßöffnung) zur Pumpe beispielsweise in der einen Anschlußwand des zylindrischen Innenraumes oder in der Nähe dieser Abschlußwand, der Anschluß (d.h. die Durchlaßöffnung (zur Manschette etwas beabstandet davon mehr zur Mitte des zylindreschen Innenraumes und der Anscluß (d.h. die Durchlaßöffnung) zur Atmosphäre in der anderen Abschlußwand oder zumindest näher zu dieser etwas im Abstand vom Anschluß zur Manschette in der Mantelwandung des zylindrischen Innenraums angeordnet sein. In der ersten Stellung ist der dichtende Körperanschnitt im Zwischenraum zwischen dem rand der Anschlußöffnung der Manschette und dem Rand der Anschlußöffnung zur Atmosphäre angeordnet und durch seine dichtende und gleitende Verschiebung in den Bereich zwischen Anschlußöffnung zur Pumpe und zur Manschette werden diese gegeneinander abgedichtet und die Verbindung zwischen Anschluß zur Manschette und zur Atmosphäre freigegeben. Somit ergibt sich insgesamt eine konstruktiv besonders einfache und dennoch höchst wirksame Weiterbildung der Erfindung.

Bevorzugt ist der dichtende Bersich als O-Ring, der in einer Umfangsnut des zylindrischen Steuerkörpers gehalten ist, ausgebildet. Dies hat insbesondere den Vorteil, daß gängige Teile die Bestandteile des erfindungsgemäßen Ablaßventiles bilden.

Besonders bevorzugt wird der Steuerkörper durch Reibschluß des Dichtungselementes in seiner jeweiligen Stellung gehalten. Durch die entsprechende Ausbildung und Abstimmung, die beispielsweise bei einem Messinggehäuse und einem Gummi-O-Ring passender Abmessung gegeben ist, ist es überflüssig, etwa weitere Elemente zum Halten des Ablaßventiles in seinen jeweiligen Endstellungen vorzusehen. Selbstverständlich ist der Reibschluß so zu bemessen, daß er durch den Pumpendruck bzw. durch eine geeignete Betätigung in die entgegengesetzte Richtung ohne Schwierigkeiten zu überwinden ist.

Bevorzugt ist das Ablaßventil manuell in die stabile Schnellablaßstellung überführbar. Dies entspricht der üblichen Vorgehensweise bei im wesentlichen handbetätigten Blutdruckmeßgeräten. Dabei weist der zylindrische Steuerkörper bevorzugt eine von außerhalb des zylindrischen Hohlkörpers betätigbare Handhabe zumindest zur Verstellung in die Schnellablaßstellung auf. Dadurch kann am Schnellablaßventil selbst der zylindrische Steuerkörper in die Schnellablaßstellung entgegen der Stellung, in der er durch den Pumpendruck übergeführt wird, verschoben werden. Dabei ist zweckmäßig die Handhabe als Verlängerung des zylindrischen Steuerkörpers ausgebildet und durchragt die von der Pumpe angewandte Endwand des Gehäuses mit im wesentlichen zylindrische Innenraum, so daß beispielsweise durch Daumendruck auf diese Verlängerung die Schnellablaßstellung eingestellt werden kann.

Besonders bevorzugt weist das Ablaßventil mit Schnellablaßfunktion eine Einrichtung zu Normalablaß auf. Dies hat den Vorteil, daß keine zusätzlichen Verbindungen von der Manschette zum Normalablaß vorgesehen sein müssen und die zum Ablaß dienenden Vorrichtungen insgesamt kompakter ausgebildet sein können.

Besonders bevorzugt weist die Normalablaßrichtung eine Kammer, deren Innenraum mit dem Anschluß zur Manschette verbunden ist, und ein zumindest teilweise in der Kammer befindliches Hohlteil mit elastischen Wandungen auf, wobei in mindestens einer der in der Kammer befindlichen Wandungen ein Schlitz vorgesehen ist und die Kammer im übrigen dichtend geschlossen ist und wobei der Innenraum des Hohlteils mit der Atmosphäre verbunden ist, und weist ein die Öffnung des Schlitzes beeinflussendes Element auf. Diese Element ist von außen steuerbar verstellbar ausgebildet. Die Vorzüge dieser Ausbildung sind im deutschen Patent 31 17 546 ins einzelne gehend beschrieben. Hierauf wird bezüglich der Offenbarung ausdrücklich verwiesen.

Bevorzugt ist dabei das Holteil mit elastischen Wandungen im Inneren des zylindrischen Körpers angeordnet und die Schlitze stehen mit einer Öffnung im zylindrischen Körper in Verbindung, welche Öffnung sich in dem Teil des zylindrischen Körpers befindet, der durch die Dichtung gegen die Atmosphäre abgedichtet ist. Durch diese Maßnahme ist es möglich, ein Ablaßventil sowohl mit Schnellablaß- wie mit Normalablaßfunktion zu schaffen, das äußerst kompakt und einfach aufgebaut ist und somit nur wenig Raum einnimmt, leicht ist und als einziges Element in der Verbindung zwischen Pumpe und Manschette ausreicht, wobei außer dem Manometer weitere Teile nicht erforderlich sind. Der zylindrische Körper braucht in seiner äußeren Form nicht verändert zu werden, da die den Normalablaß steuernden Bestandteile wegen ihres besonders einfachen Aufbaus in seinem Inneren vorgesehen sein können.

Besonders bevorzugt ist der Anschluß an die Atmosphäre als an der dem Pumpenanschluß abgewandten, offenen Endfläche des Gehäuses angebrachte Kappe ausgebildet, durch eine Öffnung derselben sich eine Verlängerung geringeren Durchmessers des zylindrischen Körpers nicht dichtend erstreckt, so daß die Öffnung als Luftauslaß, die Kappe als Anschlag für den zylindrischen Körper und die Verlängerung als Handhabe zum Verschieben des zylindrischen körpers in die Schnellablaßstellung dient. Dadurch weird erreicht, daß bei der Verschiebung in die erste Stellung kein besonderer Anschlag vorgesehen sein muß und andererseits die Handhabe griffgünstig am Ablaßventil angeordnet ist, so, daß sie durch einfachen Daumendruck durch die Kappe in den Innenraum hineinverschiebbar ist und damit auch den zylindrischen Steuerkörper in die Schnellablaßstellung verschiebt, Gleichzeitig kann dabei der Spalt zwischen der (zweckmäßig zentrisch angeordneten) Verlängerung und dem Rand der Öffnung in der Kappe als Luftauslaß für den zweiten Teilraum dienen. Dieser Luftauslaß kann jedoch in der beschriebenen Weise auch im Mantel des zylindrischen Innenraums des im wesentlichen zylindrischen Gehäuses vorgesehen sein. Auch bei der Verstellung des zylindrischen Steuerkörpers in die Schnellablaßstellung braucht kein gesonderter Anschlag vorgesehen zu sein, der zylindrische Körper kann in dieser Stellung gegen die der Pumpenöffnung nächstgelegene eine Endwand des zylindrischen Innenraums anliegen.

Besonders bevorgzugt ist die Verlängerung gegenüber dem zylindrischen Körper verstellbar ausgebildet und verstellt das die Öffnung des Schlitzes beeinflussende Element. Bezüglich diese Elementes wird wieder auf das deutsche Patent 31 17 546 verwiesen. Durch diese Lösung im Rahmen der vorliegenden Erfindung ergibt sich ein besonders einfacher Aufbau, da die Handhabe somit eine Doppelfunktion ausüben kann. Je nach dem Benutzerkreis (fachkundige (Benutzer oder die Patienten selber) kann dabei die Handhabe die erwähnte Kappe so weit überragen, daß das Verstellglied des Einstellelementes für den Normalablaß stets, oder doch zumindest in der Aufpump- und Normalablaßstellung überragt oder nur soweit überragt, daß beispielsweise ohne Aufschrauben der Kappe eine Verstellung nicht vorgenommen werden kann. Zur Verstellung von der Schnellablaßstellung in die andere stabile Stellung reichen an sich sehr kleine Wegstrecken aus, die Anschlußlöcher, die sehr klein ausgebildet sein können, wegen der besonders vorteilhaften Abdichtung durch einen schmalen O-Ring auch sehr eng beieinander liegen können. Ein nur geringfügiges Herausragen der Handhabe hat wiederum den Vorteil, daß eine unbeabsichtigte Verstellung noch unwahrscheinlicher ist.

Bei der vorstehend geschilderstn Ausführungsform ist also ein Ablaßventil durch eine Verschiebung eines, in besonders bevorzugter Ausführungsform im wesentlichen kreiszylindrischen, ersten Verstellelementes, nämlich des zylindrischen Steuerkörpers, der von Normalablaß auf Schnellablaß und zurück umstellt, in einem Ventilgehäuse mit im wesentlichen kreiszylindrischen Innenraum aus einer ersten, stabilen Stellung (nämlich der Normalablaßstellung) in eine stabile Schnellablaßstellung umstellbar. Ferner ist dabei die Meß-Ablaßgeschwindigkeit, also die Ablaßgeschwindigkeit beim Normalablaß, durch Verdrehen eines zweiten Verstellelementes, vorstehend meist als Verstellglied oder Einstellelement für den Normalablaß bezeichnet, relativ zum ersten Verstellelement um die Zylinderachse des letzteren einstellbar.

Bei einem solchen Ablaßventil ist wegen der Rotationssymmetrie der Teile nicht sichergestellt, daß insbesondere das erste Verstellelement nicht beim Einstellen des zweiten Verstellelementes mitläuft, und so eine Einstellung, oder zumindest eine genaue Einstellung verhindert.

Aus der US—A—3 030 945 ist ein Ablaßventil mit Schnellablaßfünktion, für ein Blutdruckmeßgerät mit einer Pumpe zum Aufpumpen einer Manschette bekannt, das aus einer ersten, stabilen Stellung in eine stabile Schnellablaßstellung überführbar ist, wobei das Ablaßventil auch durch die Betätigung der Pumpe in die erste, stabile Stellung übergeführt wird. Das Ablaßventil wird durch Verschieben der Pumpe in Richtung auf das Ventil in die erste Stellung überführt. Nachteilig ist dabei, daß zur Überführung des Ablaßventils in die erset Stellung eine weitere Betätigung (Verschieben der Pumpe in Richtung auf das Ventil) durchgeführt werden muß, die vergessen werden kann.

Dadurch, daß an der Innenwand des Innenraums sin, in Umfangsrichtung im wesentlichen unverschiebliches, Führungselement angeordnet ist, das in eine im Außenmantel des kreiszylindrischen ersten Verstellelementes ausgebildete Axialnut eingereift, wird erreicht, daß auch bei kreiszylindrischer Ausbildung des ersten Verstellelementes und Drehung des zweiten Verstellelementes eine sichere Verstellung des zweiten Verstellelementes zur Regulierung der Meß-Ablaßgeschwindigkeit möglich ist.

Für die Funktion des zweiten Verstellelementes wird ferner, ausdrücklich auch wegen der Öffenbarung, auf das deutsche Patent 31 17 546 verwiesen.

Unter "Axialnut" ist eine Nut in Richtung (also parallel zu) der Zylinderachse des Innenraumes zu verstehen. Die Umfangsrichtung liegt in einer Ebene senkrecht zu dieser Axialrichtung.

Es kann also insbesondere auch bei kreiszylindrischer Ausbildung des ersten Verstellelementes und des zylindrischen Innenraumes, die durch einfache Herstellung (z.B. Spanabhebende Fertigung wie Drehen oder Bohren) besonders vorteilhaft ist, die sichere Einstellung des zweiten Verstellelementes auch dann gewährleistet werden, wenn diese durch eine Verdrehung erfolgt.

Besonders bevorzugt ist die Axialnut in Richtung der Verschiebung in die Schnellablaßstellung abgeschlossen, so daß der Abschluß als Anschlag bei einer Verschiebung in die erste stabile Stellung dient. Ein solcher Abschluß kann einfach dadurch bewerkstelligt werden, daß die Nut vor dem Ende des Zylindermantels endet. Dies ist bevorzugt. Er kann jedoch auch dadurch erreicht werden, daß quer zur Nut ein Element vorgesehen ist, — z.B. der dichtende O-Ring des ersten Verstellelementes, der das erste Verstellelement in Umfangsrichtung umläuft und so den Innenraum des Ventilgehäuses in zwei luftdicht abgeschlossene Teilräume unterteilt, — das die Nut quert.

Dadurch kann verhindert werden, daß das erste Verstellelemnet, das gewöhnlich durch einem Pumpenstoß zur Manschette des Blutdruckmeßgerätes in die erste stabile Stellung übergeführt wird, über die vorgesehen erste stabile Stellung hinaus gerät.

Bevorzugt weist das zweite Verstellelement über einen Teil seiner axialen Länge einen Polygonquerschnitt, vorzugsweise hexagonal, auf und ist in der Schnellablaßstellung zumindest ein Teil der axialen Länge mit Polygonquerschnitt im Berich des Führungselementes, wobei die Umfangslinie des Polygonquerschnitts so bemessen ist, daß das Führungselement eine Drehung des zweiten Verstellelements relativ zum Ventilgehäuse nur um einen kleinen Winkel erlaubt, während in der ersten stabilen Stellung der Teil der axialen Länge mit Polygonquerschnitt außerhalb des Bereichs des Führungselementes liegt.

Dies hat insbesondere den Vorteil, daß auf einfache Weise erreicht wird, daß eine Verstellung der Meß- oder Normalablaßgeschwindigkeit nur dann möglich ist, wenn sich das erste Verstellelement in der ersten stabilen Stellung befindert. In dieser Stellung ist die Manschette normalerweise aufgepumpt und eine Regulierung der Meß-Ablaßgeschwindigkeit ggfs. erforderlich. Nach dem Schnellablaß wird das Blutdruckmeßgerät normalerweise beiseitegelegt, und es ist erwünscht, daß dann auch keine unbeabsichtige Verstellung der Meß-Ablaßgeschwindigkeit vorgenommen wird, die sich etwa ein regelmäßiger Benutzer eingestellt hat.

Unter einem kleinen Winkel soll ein solcher Winkel verstanden werden, der die Meß-Ablaßgeschwindigkeit nicht wesentlich verändert. Das hängt selbstverständlich von der besonderen Ausbildung des jeweiligen Gerätes, z.B. von der Steigung eines Gewindes ab, das das erste Verstellelement mit dem zweiten Verstellelement verbindet. Bei einem hexagonalen Polygonquerschnitt, der besonders bevorzugt ist, weil hexagonale Elemente, bspw. als Schraubenköpfe oder Muttern, serienmäßig vorliegen, wird der maximale Verstellwinkel, den das Führungselement im Bereich der axialen Länge mit Polygonquerschnitt noch erlaubt, bspw. 60° betragen, da dann die nächste Ecke des Sechskantes an das Führungselemente anstößt.

Bevorzugt ist das Führungselement als U-förmig gebogenes Drahtstück ausgebildet, das am Ventilgehäuse derart angebracht ist, daß sein einer Schenkel in axialer Richtung entlang der Innenwand des zylindrischen Innenraumes in diesen hineinragt, daß die U-förmige Umbiegung die Umfangswand des zylindrischen Innenraumes zumindest teilweise überspannt, und sein anderer Schenkel, in der Umfangswand, vorzugsweise in einer axialen Bohrung, oder an der Außenseite der Umfangswand, gegen eine Verschiebung in Umfangsrichtung festgelegt ist.

Diese Ausbildung hat insbesondere den Vorteil, daß das Führungselement einfach herzustellen und leicht an dem Ventilgehäuse anbringbar und weider davon lösbar ist. Der eine Schenkel greift dann in die Axialnut des ersten Verstellelementes ein und sein Ende kommt in der ersten stabilen Stellung ggfs. mit dem Anschlag in der Axialnut in Eingriff. Durch den Eingriff des anderen Schenkels in eine axiale Bohrung in der Umfangswand (im Ventilgehäuse) ist sichergestellt, daß das als Führungselement wirkende Drahtstück in Umfangsrichtung im wesentlichen unverschieblich ist, es kann aber ein gewisses Spiel eingebaut werden, Dadurch, daß die U-förmige Umbiegung die Umfangswand überspannt, kann ein Anschlag bspw. für eine Polygonecke des zweiten Verstellelementes geschaffen sein, das in der Schnellablaßstellung hinreichend weit in Richtung des zylindrischen Innenraumes verschoben sein muß.

In einer noch im einzelnen zu beschreibenden Ausführungsform ist eine den zylindrischen Innenraum in Richtung der ersten stabilen Stellung abschließende Kappe auf dem Ventilgehäuse drehbar angebracht, durch eine Mittenöffnung derselben das zweite Verstellelement axial verschieblich hindurchragt. Diese Kappe ist jedoch auf die Umfangswandung des Innenraumes aufgeschraubt und dient nur als Anschlag für das erste Verstellelement. Die "Drehbarkeit" dient also nur dem Aufschrauben der Kappe, nicht aber der Einstellung. Es soll aber erreicht werden, daß sie zur leichteren Einstellung des zweiten Verstellelementes beiträgt. Dies wird dadurch erreicht, daß die Mittenöffnung derart an die Querschnittsform des zweiten Verstellelementes angepaßt ist, daß das zweite Verstellelement drehfest mit der Kappe verbunden ist; und daß die Kappe axial unverschieblich ist. Während also in der einen Ausführungsform dei Kappe beim Drehen (wegen des vorgesehenen Gewindes) axial verschieblich ist, ist sie jetzt in einer besonders bevorzugten Ausführungsform drehbar, aber axial unverschieblich, angebracht. Ferner ist die Querschnittsform des zweiten Verstellelementes derart an die Mittenöffnung angepaßt, daß sich das zweite Verstellelement nicht mehr um einen nennenswerten Winkel relativ zur Mittenöffnung drehen läßt. Dadurch wird andererseits auch durch Drehung der Kappe das zweite Verstellelement mitgenommen, so daß ein größerer Radius für den Angriff der einstellenden Hand zur Verfügung steht und somit auch eine feinere Einstellung möglich ist. Die Querschnittsform kann bspw. hexagonal sein, wodurch einerseits in der Schnellablaßstellung ein Eingriff mit dem Führungselement sichergestellt sein kann und andererseits auch eine einfache Form, für die Herstellungs-Werkzeuge regelmäßig zur Verfügung stehen, für die Mittenöffnung gegeben ist.

Bevorzugt ist eine Winkelbegrenzung für die Verdrehung der Kappe vorgesehen. Dadurch kann festgelegt werden, in welchem Bereich eine Verstellung des zweiten Verstellelementes möglich sein soll.

Bevorzugt ist in der Seitenwand der Kappe eine Umfangsnut ausgebildet, die zumindest teilweise zu mindestens einem ins Innere der Kappe offenen Schlitz ausgebildet ist, ist in der Umfangswandung des zylindrischen Innenraums unter der Kappe eine entsprechende Nut ausgebildet, und ist ein Befestigungselement vorhanden, das in die Nut der Kappe und durch den Schlit in die Nut in der Umfangswand des Innenraums eingreift. Unter Seitenwand der Kappe wird die (zylindrische) Wand in Axialrichtung verstanden. Durch die beanspruchte Ausbildung ist es möglich, die Kappe drehfest, jedoch axial unverschieblich auf der, das Ventilgehäuse ggfs. etwas überragenden, Umfangswand des kreiszylindrischen Innenraums im Ventilkörper zu halten. Das Befestigungselement läuft dann in der Nut in der Außenseite der Umfangswand und ermöglicht so eine Drehung der Kappe.

Bevorzugt ist das Befestigungselement als, im wesentlichen ringförmiger, Draht ausgebildet, von dem ein Teil seiner Umfangslänge so zur Mitte des Rings aus- oder umgebogen ist, daß er durch den Schlitz in die Wandungsnut eingreift.

Der ringförmige Draht liegt also im wesentlichen in der Umfangsnut der Kappe, durchragt den Schlitz bspw. mittels einer Einbuchtung, die ihrerseits wieder soweit vorsteht, daß sie in die Nut in der Außenseite der Umfangswandung des zylindrischen Innenraums eingreift.

Zum Zwecke der erleichterten Anbringung des ringförmigen Drahtes ist dieser bevorzugt unterbrochen, die Enden können ebenfalls zur Kreismitte hin abgebogen sein und durch einen Schlitz der Kappe in die Außenumfangsnut des zylindrischen Innenraums eingreifen.

Bevorzugt ist in der Wandungsnut ein Anschlag für das in sie eingreifende Befestigungselement angeordnet. Dadurch kann die Drehung der Kappe relative zur Umfangswandung des zylindrischen Innenraumes nach Wunsch begrenzt werden.

Bevorzugt durchdringt das U-förmig gebogene Drahtstück die Wandungsnut in Axialrichtung und dient als der Anschlag. Dadurch braucht kein gesonderter Anschlag in der Wandungsnut ausgebildet zu werden, sondern das ohnehin vorgesehene Führungselement dient auch als Anschlag für die Verdrehung der Kappe.

Bevorzugt weist der mindestens eine Schlitz in der Seitenwand der Kappe eine in Umfangsrichtung größere Ausdehnung als die ihn durchgreifenden Teile des Befestigungselementes auf. Dies hat insbesondere den Vorteil, das eine Verdrehung der Kappe auch um einen Winkel größer als 360° möglich ist. Wäre das Befestigungselement gegenüber der Kappe festgelegt, und ein Anschlag vorgesehen, so wäre nur eine Drehung um maximal 360° möglich. Kann sich aber das Befestigungselement in einem größeren Schlitz noch bewegen, kann die Kappe, maximal um die Differenz zwischen Schlitzlänge und Umfangsausdehnung des Befestigungselementes, weitergedreht werden. Im Falle mehrerer Schlitze muß natürlich darauf geachtet sein, daß die Teile relativ zu den Schlitzen und zueinander so angeordnet sind, duß die Kappe relativ zum Befestigungselement verdrehbar ist. Ebenso müssen, wie gesagt, alle Schlitze die nötige Ausdehnung in Umfangsrichtung haben.

Nachfolgend wird die Erfindung anhand bevorzugter Ausführungsformen unter Bezugnahme auf die beigefügten Zeichnungen, auf die wegen ihrer großen Klarheit und Übersichtlichkeit bezüglich der Offenbarung ausdrücklich verwiesen wird, noch näher erläutert.

Es zeigen:

Fig. 1 eine schematische Gesamtansicht eines Blutdruckmeßgerätes mit einem Ablaßventil gemäß der Erfindung;

Fig. 2 eine noch mehr vergrößert dargestellte Schnittansicht des Ablaßventils gemäß der Erfindung in seiner ersten Arbeitslage zum Aufpumpen der Armmanschette für den Blutdruckmeßvorgang und für den Normalablaß;

Fig. 3 eine ebenfalls vergrößert dargestellte Schnittansicht des Ablaßventils nach Fig. 2 in seiner zweiten Arbeitslage für den Schnellablaß des Druckes aus der Armmanschette;

Fig. 4 eine teilweise Seitenansicht einer weiteren Ausführungsform des Ventilgehäuses, teilweise im Schnitt;

Fig. 5 das erste Verstellelement, teilweise im Schnitt;

Fig. 6A eine andere Ausführungsform der Kappe von unten;

Fig. 6B eine Seitenansicht der Kappe aus Fig. 6A. teilweise im Schnitt;

Fig. 7 einen Ausschnitt aus Fig. 4, im Schnitt, mit eingelegtem Befestigungselement;

Fig. 8 eine Aufsicht auf ein als Befestigungselement dienendes Drahtstück;

Fig. 9 eine Seitenansicht eines U-förmigen, als Führungselement dienenden Drahtstücks;

Fig. 10A eine Ansicht von oben einer anderen Ausführungsform des zweiten Verstellelementes;

Fig. 10B eine, teilweise geschnittene, Seitenansicht des zweiten Verstellelements aus Fig. 10A;

Fig. 11 das Ablaßventil gemäß der weiteren Ausführungsform im Zusammenbau, im Schnitt.

Gleiche Bezugszeichen bezeichnen auch in unterschiedlichen Ausführungsformen gleiche oder entsprechende Teile.

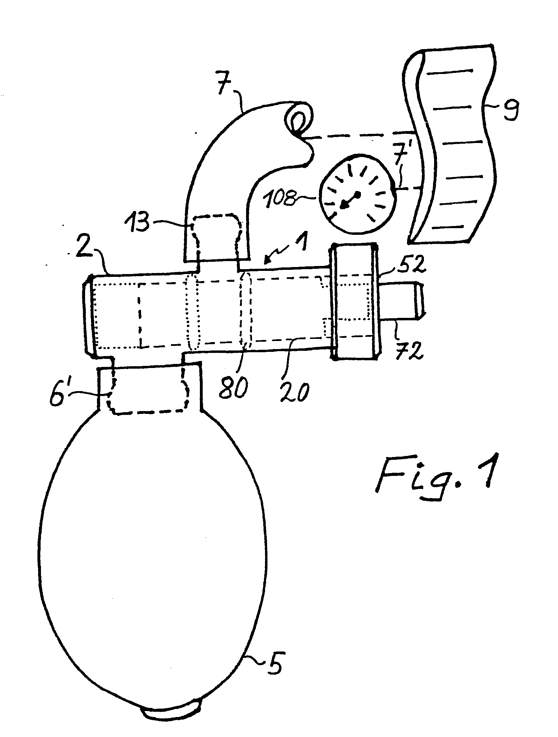

In Fig. 1 ist die einfache Gesamtanordnung zu erkennen, die durch das hier beschriebene Ablaßventil möglich ist, wobei das Grundprinzip der hier beschriebenen Ausführungsform an den beiden gestrichelt dargestellten Lagen eines Ventilelements erkennbar ist.

Gemäß Fig. 1 weist das Schnellablaßventil 1 ein im wesentlichen zylindrisches Gehäuse 2 mit einer ersten Anschlußolive 6' und einer zweiten Anschlußolive 13 auf. Auf die Anschlußolive 6' ist ein Gebläseball 5 aus Gummi aufgeschoben. Auf die Anschlußolive 13 ist ein zur Armmanschette 9 führender flexibler Schlauch 7 aufgeschoben. Mit der Armmanschette 9 ist über einen flexiblen Schlauch 7' ein Manometer 108 verbunden. Das Schnellablaßventil 1 weist einen Betätigungsknopf 72 zur Umschaltung aus seiner Auspump- und Blutdruckmeßlage in seine Druckschnellablaßlage auf.

In unterbrochenen Umrissen sind im Inneren des Gehäuses 2 die beiden Stellungen eines zylindrischen Steuerkörpers 20, an dem axial unverschieblich eine ebenfalls nur angedeutete O-Richtdichtung 80 befestigt ist, angedeutet. Durch die Dichtung 80 wird der Innenraum des Gehäuses 2 in zwei (veränderliche) Teilräume unterteilt. In der gestrichelt dargestellten, in Fig. 1 rechten Stellung des zylindrischen Steuerkörpers 20 sind an den in Fig. 1 linken Teilraum sowohl die Pumpe 5 über den Anschluß 6' als auch die Manschette 9 über den Anschluß 13 verbunden. Da dieser Teilraum im übrigen durch die Dichtung 80 abgedichtet ist, kann ein normales Aufpumpen der Manschette erfolge Zum Schnellablaß wird durch Druck auf den Betätigungsknopf 72 der zylindrische Steuerkörper 20 in die Lage, die durch gepunktete Teilumrisse angedeutet ist, verschoben. Dadurch wird der linke Teilraum derart verkleinert, daß nur noch der Anschluß von der Pumpe in ihn führt. Nunmehr führt der Anschluß von und zur Manschette in den rechten Teilraum, der (durch 52 angedeutet) mit der Atmosphäre in Verbindung steht. Dadurch kann die Luft aus der Manschette frei in die Atmosphäre strömen, was als Schnellablaß wirkt. Eine erneute Betätigung der Pumpe 5 erhöht den Druck im linken, verkleinerten Teilraum so weit, daß der zylindrische Körper mit seinem Dichtungselement in die gestrichelt gezeigte Lage zurückverschoben wird, wodurch die Manschette dann weiter aufgepumpt werden kann.

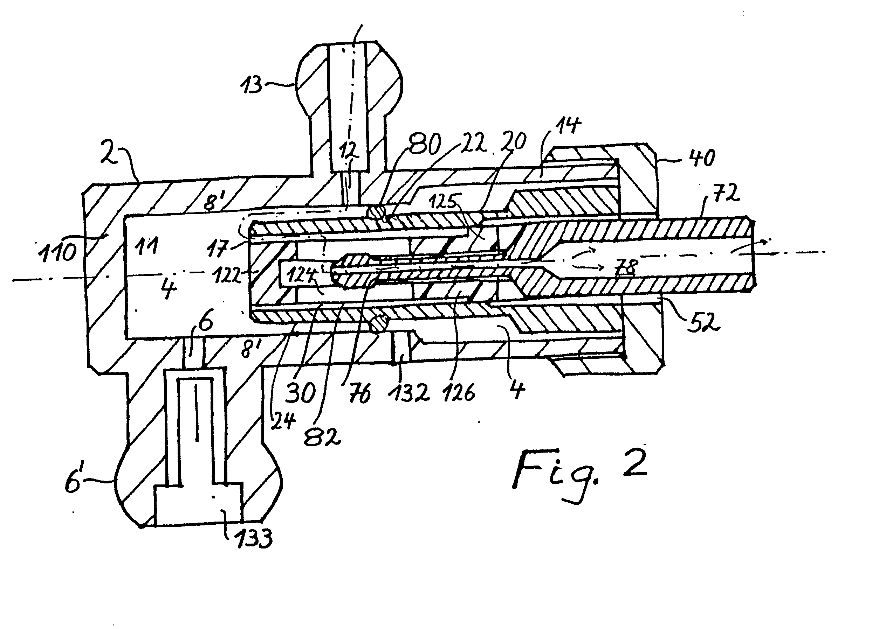

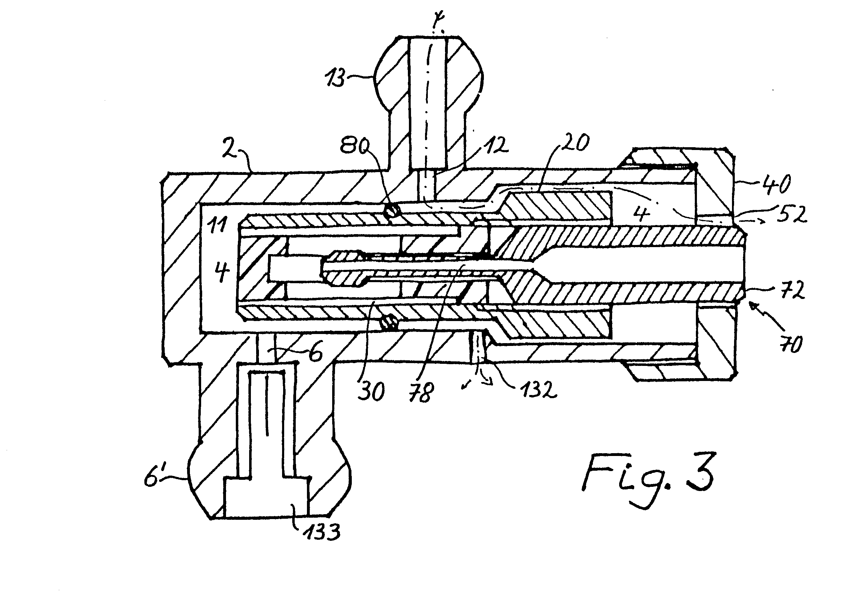

In Fig. 2 und 3 ist dieser Grundaufbau näher im Detail zu erkennen.

Das Gehäuse 2 des Ablaßventil 1 ist hohlzylindrisch und weist an seiner linken Seite (in den Fig. gesehen) einen Boden 110 auf, der es an dieser Seite verschließt. Der zylindrische Innenraum 4 des Gehäuses 2 ist, gesehen in den Fig. 2 u. 3 von links nach rechts einmal abgestuft. In den Figuren links gesehen befindet sich ein erster Wandabschnitt 8' mit kleinerem Durchmesser des Innenraums, in welchen von links gerechnet zuerst eine Öffnung 6 von der Anschlußolive 6', dann eine Öffnung 12 von der Anschlußolive 13 und dann eine Öffnung 52 zur Atmosphäre, mündet. An den Wandabschnitt 8' schließt sich nach rechts (in der Fig. gesehen) ein Wandabschnitt 14 mit größerem Durchmesser des Innenraums an.

In den Innenraum 4 des Gehäuses ist ein Druckablaßventil eingesetzt, das als Ganzes als Steuerkörper 20 für die Schnellablaßfunktion dient. Das Druckablaßventil 20 weist einen im wesentlichen zylindrischen, an den Axialenden offenen Hauptkörper auf. Der Hauptkörper besteht, ebenso wie das Gehäuse 2, im wesentlichen aus Metall (z.B. Messing) oder einem festen Kunststoff. In einer Nut 22 am Außenumfang des zylindrischen Hauptkörpers, die den Hauptkörper im Ausführungsbeispiel der Fig. 1—3 in Umfangsrichtung etwa in der Mitte seiner Axialerstreckung umgibt, ist ein O-Ring 80 eingesetzt. Der Außenendurchmesser des zylindrischen Hauptkörpers von 20, der Umfangsnut 22 und des O-Rings 80 sind so gewählt, daß der O-Ring in dichtender Anlage an dem Innenwandanschnitt 105 des Gehauses 2 anliegt.

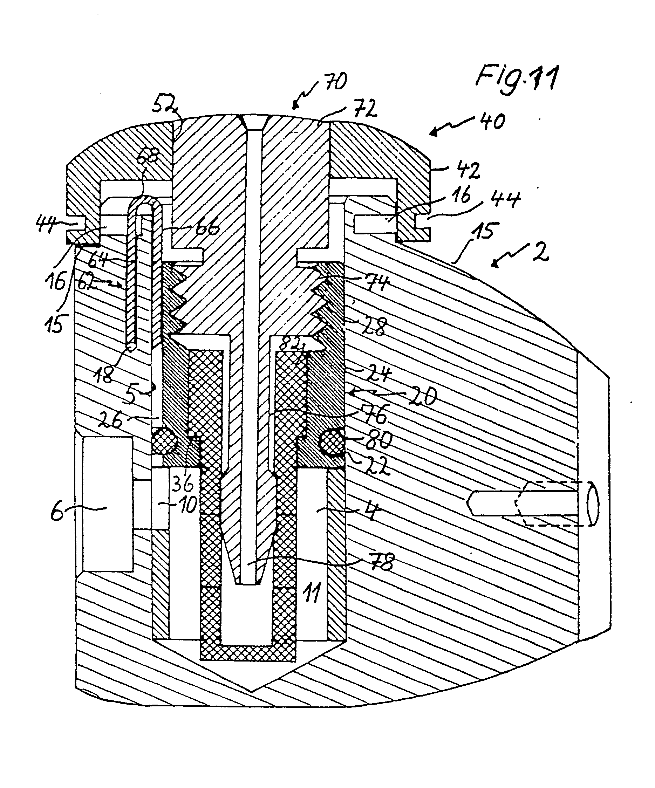

Der Hauptkörper von 20 ist hohlzylindrisch. In die Innenbohrung 30 des Hauptkörpers ist ein mit einem Boden 122 versehener hohlzylindrischer Einstellkörper 82 aus gummi-elastischem Material, vorzugweise Gummi, eingesetzt, der in seiner Zylinderwand zwei Schlitze 124 aufweist. Der hohlzylindrische Einstellkörper dieser Ausführungsform sitzt mit seinem in den Fig. 2 und 3 gesehen rechten Umfangsteil 125 mit größerem Durchmesser, der einen etwa größeren Durchmesser als der Durchmesser der Innenbohrung 30 des zylindrischen Hauptkörpers von 20 besitzt, durch Reibschluß sicher in der Innenbohrung 30 des Hauptkörpers. Der (in den Fig. 2 und 3 gesehen) linke Umfangsteil 126 weist einen etwas kleineren Durchmesser als die Innenbohrung 30 des zylindrischen Hauptkörpers der Fig. 2 und 3 auf, so daß Luft aus dem links der Dichtung 80 befindlichen ersten Teilraums 11 des zylindrischen Innenraums 4 durch die offene Endlfäche des Hauptkörpers von 20 in den entsprechenden Zwischenraum zwischen dem linken Umfangsteil 126 des hohlzylindrischen Einstellkörpers 82 und der Außenwand der Innenbohrung 30 des zylindrischen Hauptkörpers bis zu den Schlitezn 124 im Umfangsteil 126, jedoch nicht am Umfangsteil 125 mit größerem Durchmesser vorbei, strömen kann. Es kann, wie aus Fig. 11 ersichtlich, der zylindrische Hauptkörper, dort als erstes Verstellelement bezeichnet, auch, in Fig. 2 und 3 gesehen links, kürzer ausgebildet sein, so daß das Element 82 frei in den linken Teilraum 11 ragt.

Der Betätigungsknopf 72 ist in den (in Fig. 2 und 3 gesehen) rechten Teil des zylindrischen Hauptkörpers verstellbar eingeschraubt (gestrichelt angedeutet) und weist einen sich in den hohlzylindrischen Einstellkörper 82 erstreckenden länglichen Fortsatz auf, der an seinem Vorderende einen Bereich mit vergrößertem Durchmesser 76 besitzt. Durch den Einstellknopf 72 und durch seinen Fortsatz 76 erstreckt sich eine durchgehende Axialbohrung oder Kanal 78. Durch diese ist der Innenraum des hohlzylindrischen Einstellkörpers 82, in welchen Innenraum die Schlitze führen, mit der Atmosphäre verbunden.

Auf ein (gestrichelt angedeutets) Gewinde am Außenumfang des Gehäuses 2 an seinem (in den Fig. 2 und 3 gesehen) rechten Ende ist eine Kappe 40 aufgesetzt, in dieser Ausführungsform aufgeschraubt, die das Gehäuse an seiner (in den Fig. 2 und 3 gesehen) rechten Seite abschließt. In der Mitte des Deckels ist eine, in dieser Ausführungsform kreisförmige, Bohrung 52 mit einem geringfügig größeren Durchmesser als der Außendurchmesser des Betätigungsknopfes vorgesehen, durch welche der Betätigungsknopf (in den Fig. 2 und 3 gesehen) nach rechts ragt. Die Kappe 40 bildet ferner somit den Anschlag für eine nach rechts gerichtete Bewegung des die Umstellung bewirkenden zylindrischen Steuerkörpers 20.

Nachfolgend wird die Funktion des Ablaßventils beschrieben:

Wenn der Gebläseball 5 zusammengedrückt wird, so wird Luft durch das Gummiventil 133 in der Anschlußolive 6' durch die Öffnung 5 in den ersten (linken) Teilraum 11 des Innenraums 4 gepreßt. Wenn sich zu diesem Zeitpunkt das als Steuerkörper wirkende Durchlaßventil 20 in seiner in Fig. 3 gezeigten, ersten stabilen Stellung befindet, dann wird es sich unter dem Einfluß des sich im zylindrischen ersten Teilraum 11 (in den Fig. 2 und 3 gesehen) links vom O-Ring 80 aufbauenden Druckes nach rechts in die in Fig. 2 gezeigte Lage verschieben. (Dabei kann durch die Schlitze 124 nicht genügend Luft hinreichend schnell entweichen, um einen Druckaufbau zu behindern). Dabei wird die in die Abschlußolive 13 führende Öffnung 12 vom O-Ring 80 zum Druckraum 11 freigegeben.

Die mittels des Gebläseballs 5 eingepreßte Luft kann somit durch die Anschlußolive 13 in den Schlauch 7 und zur Armmanschette sowie zum Manometer 108 strömen.

Nach Beendigung der Pumpbewegung am Gebläseball 5 kann die unter Druck stehende Luft aus der Armmanschette 9 über die Anschlußolive 13, die Schlitze 124 im hohlzylindrischen Einstellkörper 82 und die Bohrung 78 zur Umgebung hin entweichen, wie dies in Fig. 2 durch die strichpunktierten Pfeile angedeutet ist. Für den Zutritt der Luft zu den Schlitzen kann in dieser Ausführungsform auch eine Öffnung im Zylindermantal 24 des Hauptkörpers 20 links vom O-Ring 80 vorgesehen sein.

Einzelheiten der Wirkungsweise des Druckablaßventils sind genauer im deutschen Patent 31 17 546 beschrieben, so daß hier eine genaue Erläuterung entbehrlich ist. Zur Einstellung des Druckablaßventils kann der in der Stellung der Fig. 2 herausragende Betätigungsknopf 72 mit einer Rändelung versehen sein.

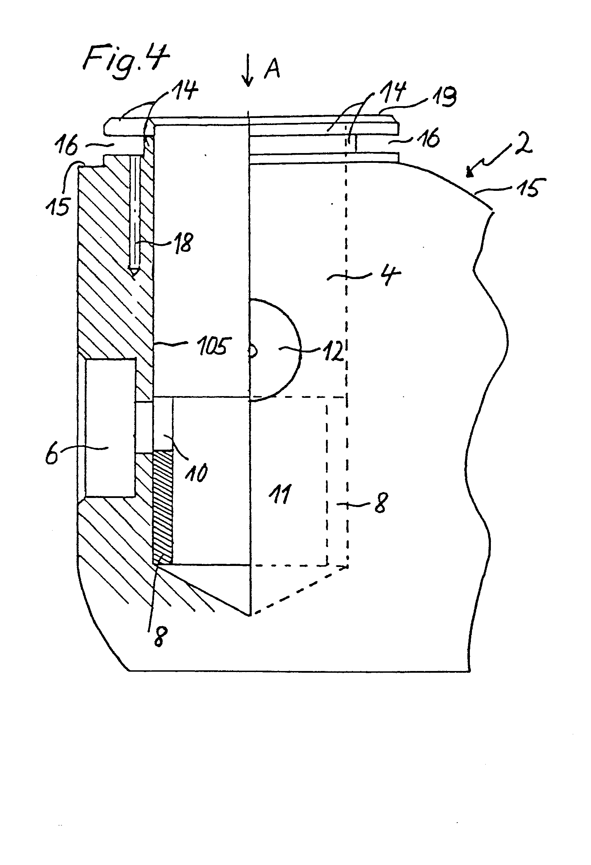

Soll der Druck in der Armmanschette 9 nach beendeter Blutdruckmessung rasch vollständig abgelassen werden, wird der Steuerkörper 20 mittels des Betätigungsknopfes 72 in die in Fig. 3 gezeigte Lage in das Gehäuse 2 eingedrückt. Dadurch wird der O-Ring in eine Lage in Fig. 3 gesehen links, in Fig. 11 gesehen unterhalb, der Öffnung 12 der Anschlußolive 13 verlagert. Im Teilraum 11 kann ein Anschlag 8 für die Verschiebung des Teiles 20 nach links vorgesehen sein, wie z.B. in Fig. 4 und 11 ersichtlich. Der Anschlag kann beispielsweise auch durch einen Ansatz am Umfang am rechten Ende des Betätigungsknopfes 72 gebildet werden oder durch die Stufe im Innendurchmesser des Gehäuses 2 im Zusammenwirken mit der Stufe im Außendurchmesser der Hauptkörpers 20, wie in Fig. 2 und 3 dargestellt. Durch diese Verschiebung kann aus der Armmanschette 9 über die Anschlußolive 13, die Öffnung 12, den Zwischenraum zwischen der Innenwand des Gehäuses 2 und der Außenwand des zylindrischen Hauptkörpers des das Druckablaßventil enthaltenden Verstellelementes 20 und die Öffnung 132 im Mantel des Gehäuses (und den Spalt zwischen dem Außenumfang des Betätigungsknopfes 72 und der Bohrung 52 in der Kappe 40) Luft der Umgebung hin abströmen, wie durch die strichpunktierten Pfeile in Fig. 3 angedeutet.

Zur Einleitung des Druckschnellablaßvorgangs reicht eine kurze Betätigung zur Verschieben des das Druckablaßventil 20 enthaltenden Körpers mit seiner O-Ring dichtung 80 in die in Fig. 3 gezeigte Lage aus. Diese Lage behält das Schnellablaßventil selbsttätig bei, so daß die Bedienungsperson diesem Vorgang keine weitere Aufmerksamkeit mehr schenken muß.

Ebenso braucht das Schnellablaßventil 1 für einen erneuten Blutdruckmeßvorgang nicht erneut betätigt zu werden, da es beim ersten Pumpstoßmuus dem Gebläseball 5 selbsttätig in die in Fig. 2 gezeigte Lage verschoben wird, in welcher sich der O-Ring in einer Lage, in Fig. 2 gesehen rechts, und in Fig. 11 gesehen oberhalb, der Öffnung 12 befindet.

In Fig. 4 weist ein insgesamt wieder mit 2 bezeichnetes Ventilgehäuse in einer weiteren Aufführungsform den im wesentlichen kreiszylindrischen Innenraum 4, der nach der in Fig. 4 oberen Seite offen ist, auf.

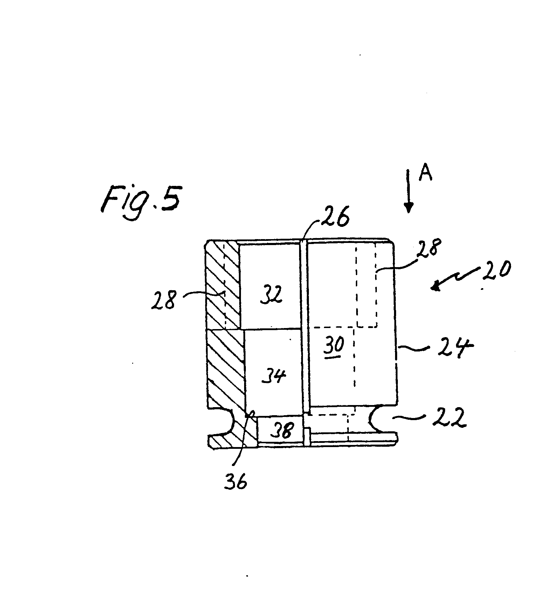

Das Ventilgehäuse hat an seiner, in Fig. 4 linken, Vorderseite eine Ausnehmung 6 zum Einsetzen des Anschlußstutzens (nicht gezeigt) für die Pumpe 5 (Fig. 1) des Blutdruckmeßgerätes. Dieser Pumpenanschluß mündet in den unteren Bereich 11 des zylindrischen Innenraumes 4, der, entweder durch einen verminderten Bohrquerschnitt, wie in Fig. 2 und 3, oder durch eine gesonderte Anschlaghülse 8, verengt ist. In der Anschlaghülse ist zum Durchtritt der Luft aus der Pumpe eine Ausnehmung 10 vorgesehen. Die Anschlaghülse 8 dient als Anschlag für das erste Verstellelement (Fig. 5) in seiner Schnellablaßstellung.

Oberhalb der Anschlaghülse 8 ist der Anschluß 12 für den Zutritt der Luft aus dem Innenraum 4 des Ventilgehäuses 2 zur Manschette (108 in Fig. 1) angeordnet. Die Innenwand des kreiszylindrischen Innenraumes ist mit 105 bezeichnet.

Die Außenwandung 14 des kreiszylindrischen Innenraumes 4 ist etwas über die in Fig. 4 obere Fläche 15 des Ventilgehäuses 2 emporgezogen. In der so entstandenen zylindrischen Umfangswand ist eine Außen-Umfangs-Nut 16 vorgesehen. In der Axialrichtung A (der Pfeil A ist in die Richtung der Umstellung in die Schnellablaßstellung gerichtet) erstreckt sich, in der radialen Entfernung der Nut 16 gelegen, eine senkrechte Bohrung 18 in das Ventilgehäuse.

Die in Fig. 5 gezeigte Auführungsform eines Steuerkörpers, nachfolgend als erstes Verstellelement 20 bezeichnet, ist ebenfalls im wesentlichen kreiszylindrisch ausgebildet. Sein Außenmantel 24 hat ein gewisses Spiel im Innenraum 4 und wird wiederum (vgl. Fig. 1 bis 3) in diesem dichtend durch einen in der Umfangsnut 22 festgelegten Gumm-O-Ring 80 (s. Fig. 11) geführt.

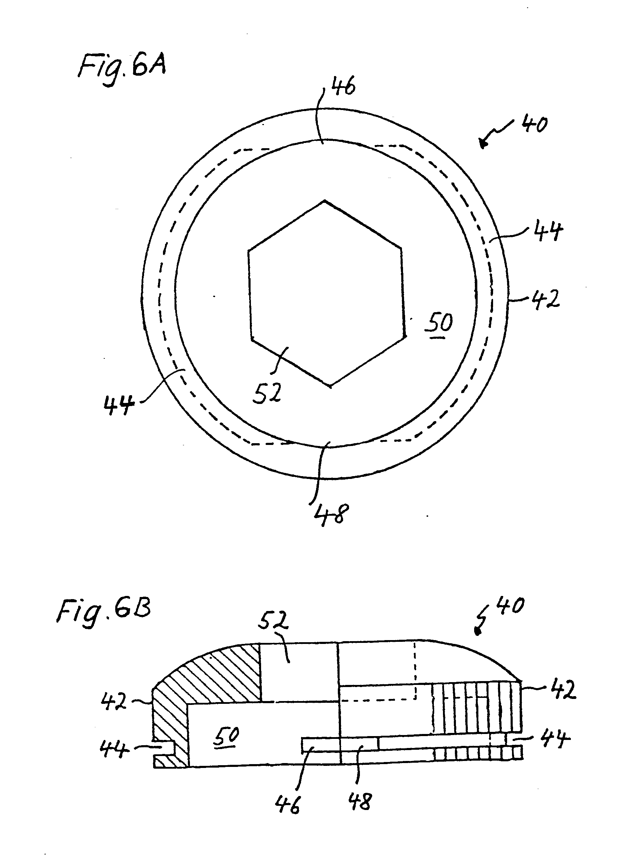

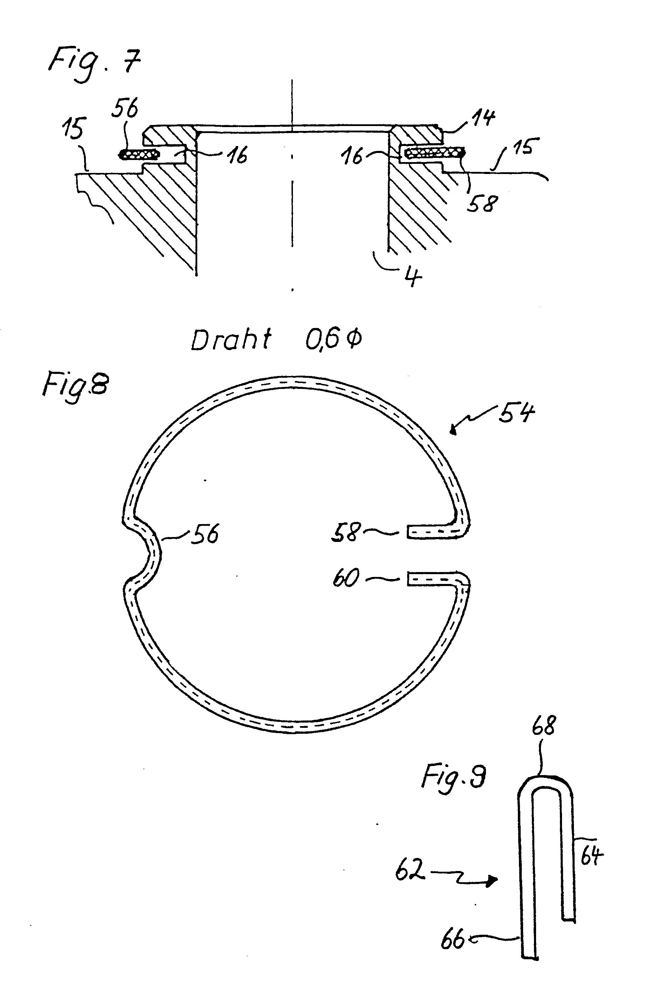

Im Außenmantel 24 des Verstellelementes 20 ist eine Axialnut 26 ausgebildet, die das noch zu beschreibende Führungselement (s. Fig. 9) aufnehmen kann. Die Bewegung des ersten Verstellelements 20 in Richtung des Pfeiles A wird durch die Anschlaghülse 8 (Fig. 4) begrenzt. Die Bewegung in die zum Pfeil A parallele, entgegengesetzte Richtung kann durch das Anstoßen des Führungselementes am unteren Ende der Nut 26 oder bspw. am O-Ring 80 in der Nut 22 begrenzt werden. Sie kann jedoch auch alternativ bevorzugt durch den Anschlag des ersten Verstellelementes, das gegen die Kappe (s. Fig. 6 und 11) läuft, begrenzt werden.

Im Inneren des ersten Verstellelementes 20 befindet sich eine konzentrischer, achsparalleler kreiszylindrischer Hohlraum 30, der nach oben und unten offen ist.

Der, in Fig. 5 gesehen, obere Abschnitt 32 des Hohlraumes ist mit einem Innengewinde 28 versehen, in das das zweite Verstellelement (s. Fig. 10B) einschraubbar ist. Im mittleren Abschnitt 34 wird das obere Teil eines elastischen Elementes 82 (s. Fig. 11) aufgenommen, das sich auf der Schulter 36 abstützt und durch den unteren Teil 38 des Hohlraumes 30 nach dem Zusammenbau in den Innenraum 4 des Ventilgehäuses ragt. Die Funktion diese elastischen Elementes zur Verstetigung des Luftablasses in der Meß-Ablaßeinstellung in im deutschen Patent 31 17 546 (dort (Bezugszeichen 30) noch näher beschrieben, so daß darauf hier nicht näher eingengen wird. Es wurde in der vorliegenden Anmeldung meist als Hohlteil bzw. Einstellkörper bezeichnet.

In Fig. 6A und 6B ist eine Kappe 40 gezeigt, deren Abmessungen so beschaffen sind, daß sie über den die Hauptoberfläche 15 des Ventilgehäuses 2 überragenden Umfangswandungsteil 14 konzentrisch paßt und sich auf der Fläche 15 abstützt. In der Seitenwand 42 ist eine äußere Umfangsnut 44 ausgebildet, die bei aufgesetzter Kappe auf der Höhe der Nut 16 in Fig. 4 liegt.

Die Nut 44 ist an im vorliegenden Beispiel zwei Stellen zu Schlitzen 46 bzw. 48 zum Innenraum 50 hin durchgebrochen und steht dadurch mit der Nut 16 in Verbindung.

In der Mitte der Kappe ist die Öffnung 52 mit hier senkrecht zur Zylinderachse hexagonalem Querschnitt angeordnet. Die Seitenwand der Kappe ist mit einer Rändelung versehen.

In Fig. 8 ist zu einem federnden Ring gebogener Draht 54 gezeigt, der als Befestigungselement für die Kappe 40 auf dem Ventilgehäuse 2 dient. Die Kappe 40 wird über den durch die Umfangswandung 14 das Innenraums 4 gebildeten Vorsprung gestülpt. Wie schon erwähnt, befindet sich dann die Nut 44 auf der Höhe der Nut 16. Der federnde Ring 54 wird aufgebogen und so in die Nut 44 eingelegt, daß sein zur Mitte ausgebogener Bereich 56 am einen Schlitz, 46, liegt, während die zur Mitte abgebogenen Enden 58 und 60 des Drahtringes 54 an die Stelle des Schlitzes 48 kommen. Der Ring, die Ausbiegung 56 und die abgebogenen Enden 58 und 60 sind so bemessen, daß der Ring mit seiner Kreislinie in der Nut 44 liegt und die Teile 56, 58 und 60 durch die Schlitze 46 bzw. 48 hindurch in die Nut 16 ragen, zweckmäßig ohne deren achsparallele Innenwand zu berühren. Fig. 7 zeigt die Führung des Drahtringes 54 durch die Teile 56, 58 in der Nut 16, wobei die Kappe 40 weggelassen wurde.

Fig. 9 zeigt ein als Führungselement für das erste Verstellelement 20 dienendes, U-förmig gebogenes Drachtstück. Der, ggfs. kürzere, Schenkel 64 des Drahtstückes wird in die Bohrung 18 eingeführt, so, daß der, ggfs, längere, Schenkel 66 der Drahtstückes 62 in den Innenraum 4 (Fig. 4) ragt, wobei das Verbindungsstück 68 in seiner Breite so bemessen ist, daß der längere Schenkel 66 mit geringem Spiel an der Innenwand 5 anliegt. Der Schenkel 66 und die Nut 26 (Fig. 5) im ersten Verstellelement 20 sind ferner so aufeinander abgestimmt, daß das Drahtsrück 66 ein Auf- und Abgleiten des Elementes 20 erlaubt, aber das Element 20 gegen Drehung um die Zylinderachse mit gerinem Spiel sichert.

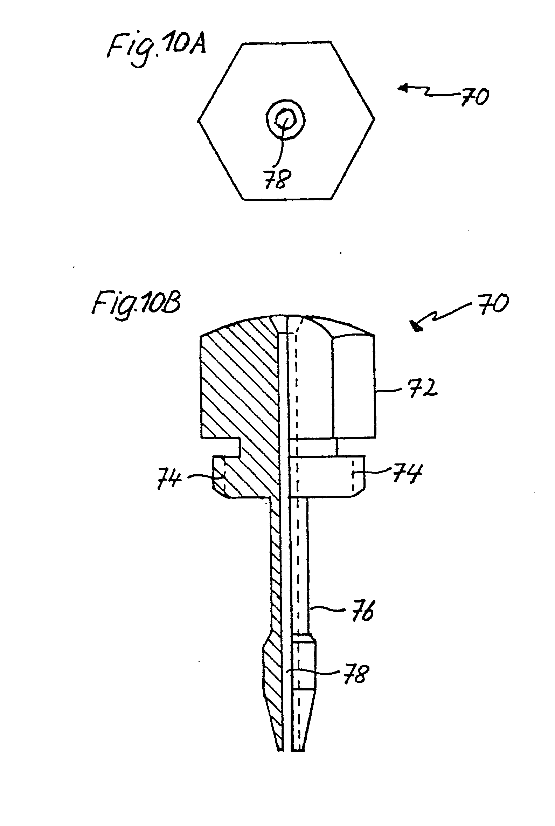

In Fig. 10B ist das zweite Einstellelement 70 gezeigt, das zur Regulierung der Meß-Ablaßgeschwindigkeit dient, wie schon vorstehend mit Bezug auf Fig. 1 bis 3 sowie im deutschen Patent 31 17 546 näher erläutert. Das zweite Verstellelement 70 weist über einen Teil seiner axialen Länge, nämlich am Kopf 72, einen polygoalen, hier hexagonalen, Querschnitt auf, wie aus Fig. 10A besonders deutlich ersichtlich. Dieser ist an dier Mittenöffnung 52 der Kappe 40 derart angepaßt, daß er in Axialrichtung gleitend hindurchpaßt, aber beim Verdrehen der Kappe ohne weiteres mitgenommen wird.

Unterhalb des Kopfes 72 ist ein Außengewinde 74 vorgesehen, das zum Innengewinde 28 der ersten Verstellelements paßt. Eine Drehung des zweiten Verstellelementes verschiebt also das zweite Verstellelement in Axialrichtung und laßt dadurch die Spreiznadel 76 des zweiten Verstellelementes tiefer oder weniger tief in das (s. Fig. 11) elastische Teil 82 im Raum 34/38/4 eindringen, wodurch die Meß-Ablaßgeschwindigkeit regulierbar ist. Die Luft kann aus dem Innenraum 4 durch den Kanal 78 im zweiten Verstellelement entweichen, was beim Aufpumpen keine Rolle spiel und für das Messen nur dann möglich ist, wenn sich der O-Ring in der Nut 22 des Ersten Verstellelementes oberhalb der Verbindungsöffnung 12 zur Manschette befindert. Wird durch Fingerdruck auf die Oberseite des Kopfes 72 das zweiten Verstellelement und mit ihm das erste Verstellelement axial in die Schnellablaßstellung verschoben, so gerät der dichtende O-Ring nach unterhalb der Öffnung 12, sdaß die Luft aus der Manschette zwischen der Außenwand 24 (Fig. 5) und der Innenwand 5 (Fig. 4) und am Kopf 72 (Fig. 10B) vorbei durch die Öffnung 52 (Fig. 6A) ungehindert ausströmen kann (Schnellablaß), wie mit Bezug auf Fig. 1 und 3 ausführlich erläutert.

Im Zusammenbau (vgl. Fig. 11) wird als das erste Verstellelement aus Fig. 5, in das das zweite Verstellelement aus Fig. 10B eingeschraubt ist, zweckmäßig mit dem U-förmigen Drahtstück aus Fig. 9 zusammen in den Innenraum 4 aus Fig. 1 eingesetzt und dort durch den in der Nut 22 liegenden O-Ring 80 dichtend geführt. Die Kappe 40 aus den Fig. 6 wird aufgesetzt und mit Hilfe des Drahtringes 58 (Fig. 7 und 8, in Fig. 11 nicht gezeigt) am Ventilgehäuse 2 festgelegt. Wie insbesonere aus Fig. 7 ersichtlich ist, ragt der bogenförmige Vorsprung 56 am Draht 54 in die Nut 16 nur so weit vor, daß er am Schenkel 64 des U-förmigen Drahtstückes (62), welcher Schenkel in der Bohrung 18 steckt und die Nut 16 durchragt, unbehindert vorbeigeschoben werden kann. Die rechtwinkeligen Abbiebungen 58 und 60 am Drahtende stoßen jedoch am Schenkel 64 an, so daß sich eine Drehbegrenzung für die Kappe auf (praktisch) 360° ergibt. Wenn die Schlitze 46 und 48 bei gleicher Anordnung (um 180° versetzt) entsprechend größer ausgebildet werden, kann eine Verdrehung um mehr als 360° erzielt werden.

Die Einschiebtiefe des ersten Verstellelementes 20 in der Schnellablaßstellung (Fig. 11) und die Höhe des Kopfes 72 des zweiten Verstellelements sind ferner so bemessen, daß der Kopf 72 in der Schnellablaßstellung seitlich des inneren Schenkels 66 des U-förmigen Drahtstückes 62 liegt. Bei dem Versuch einer Verdrehung stößt dann der Sechskantkopf 72 an den Schenkel 66 an, so daß in der Schnellablaßstellung eine Verdrehung verhindert wird. In der ersten stabilen, dem Meßablaß dienenden Stellung, bei der sich der O-Ring 80 in der Nut 22 im Element 20 oberhalb der Öffnung 12 (Fig. 4) befindet, kann hingegen der Kopf 72 über das obere Ende 68 des Drahtstücks 62 hinweggedreht werden.

In einer alternativen bevorzugten Ausführungsform kann ein dem Drahtring 54 entsprechender Drahtring in eine Innennut der Kappe 40, also eine Nut in der Wand 42 vom Innenraum 50 aus, auf der Höhe der gezeigten Nut 44, eingelegt werden. Der Draht sollte dann eine Stärke von etwa der doppelten Nuttiefe bezitzen, so daß er auch in die Nut 16 eingreifen kann.

Wie insbesondere aus Fig. 11 ersichtlich, ist der Durchmesser des ersten Verstellelementes 20 größer als der freie Durchmesser der Mittenöffnung 52 der der Kappe 40. Daher schlägt die obere Fläche des ersten Verstellelementes 20 bei dessen Überführung in die erste stabile Stellung an der Kappe an. Da der Abstand obere Fläche von 20/Innenfläche von 40 auch kleiner ist als der Abstand des unteren Endes des Schenkels 66 vom O-Ring 80, wird so eine Beschädigung des O-Rings vermieden. Die Nut 26 kann dann, wie gezeigt, durchgehend ausgebildet sein, was fertigungstechnisch einfacher ist.

**Patentansprüche**

1. Ablaßventil mit Schnellablaßfunktion, für ein Blutdruckmeßgerät mit einer Pumpe (5) zum Aufpumpen einer Manschette (9), das aus einer ersten, stabilen Stellung (Fig. 2) in eine stabile Schnellablaßstellung (Fig. 3, Fig. 11) überführbar ist, dadurch gekennzeichnet, daß das Ablaßventil (1) durch die Aufpumpbetätigung der Pumpe in die erste, stabile Stellung übergeführt wird.

2. Ablaßventil nach Anspruch 1, dadurch gekennzeichnet, daß das Ablaßventil (1) durch den Druck oder Sog der Pumpe (5) in die erste Stellung übergeführt wird.

3. Ablaßventil nach Anspruch 1 oder 2, dadurch gekennzeichnet, daß das Ablaßventil (1) ein Ventilgehäuse (2) mit im wesentlichen zylindrischem Innenraum (4) aufweist, an den die Verbindung (6) zur Pumpe (5), die Verbindung (12) zur Manschette (9) und die Atmosphäre angeschlossen (6', 13/52) sind, und daß im zylindrischen Innenraum ein entsprechender, im wesentlichen zylindrischer Steuerkörper (20) verstellbar angeordnet ist, der in einer ersten Stellung (Fig. 2) die Verbindung zwischen Pumpenanschluß und Manschettenanschluß unter Abdichtung gegen die Atmosphäre und in einer zweiten Stellung (Fig. 37, Fig. 11) (Schnellablßstellung) die Verbindung zwischen Manschettenanschluß und Atmosphäre unter Abdichtung gegen den Pumpenanschluß derart, daß ein Pumpendruck (oder gegebenenfalls -sog) den zylindrischen Steuerkörper in die erste Stellung (Fig. 2) verstellt, herstellt oder erlaubt.

4. Ablaßventil nach Anspruch 3, dadurch gekennzeichnet, daß die Durchlaßöffnung (60 zur Pumpe und die Durchlaßöffnung (12) zur Manschette in Axialrichtung des Innenraums (4) beabstandet angeordnet sind, daß der Steuerkörper (20) im Innenraum des Ventilgehäuses (2) in dessen Axialrichtung verschiebbar ist und den Innenraum in zwei voneinander abgedichtete Teilräume variabler Größe unterteilt, und daß der Steuerkörper in zwei Lagen verschiebbar ist, in deren ersten (Fig. 2) beide Durchlaßöffnungen (6, 12) in den ersten im übrigen abgeschlossenen Teilraum (11) münden und in der zweiten Lage (Fig. 3) die Durchlaßöffnung (6) zur Pumpe (5) in den ersten Teilraum mündet und die Durchlaßöffnung (12 zur Manschette (9) in den zweiten Teilraum mündet, der wenigstens in der zweiten Verschiebelage des Steuerkörpers zur Atmosphäre hin offen (132, 52) ist.

5. Ablaßventil nach Anspruch 3 oder 4, dadurch gekennzeichnet, daß der Anschluß (6, 6') der Pumpe (5), der Anschluß (12, 13) und die Manschette und der Anschluß (132, 52) an die Atmosphäre in dieser Reihenfolge in Axialrichtung gesehen an den Innenraum des Ventilgehäuses (2) angeschlossen sind und der zylindrische Steuerkörper (20) zumindest über einen Teil seiner Länge, der jedoch nicht größer ist als der Abstand zwischen den einander zugewandten Rändern der Anschlüsse, dichtend (80) und gleitbar mit der Innenwand des Hohlzylinders verbunden ist, wobei der dichtend Bereich (80) in der ersten Stellung zwischen dem Anschluß zur Manschette und zur Atmosphäre und in der Schnellablaßstellung zwischen dem Anschluß zur Manschette und dem Druckanschluß (6) zur Pumpe angeordnet ist.

6. Ablaßventil nach Anspruch 5, dadurch gekennzeichnet, daß der dichtende Bereich als O-Ring (30), der in einer Umfangsnut (22) des zylindrischen Steuerkörpers (20) gehalten ist, ausgebildet ist.

7. Ablaßventil nach Anspruch 5 oder 6, dadurch gekennzeichnet, daß der Steuerkörper (20) durch Reibschluß des Dichtungselementes (80) in seiner jeweiligen Stellung gehalten wird.

8. Ablaßventil nach einem der vorhergehenden Ansprüche, dadurch gekennzeichnet, daß das Ablaßventil manuell in die stabile Schnellablaßstellung überführbar ist.

9. Ablaßventil nach einem der Ansprüche 3 bis 8, dadurch gekennzeichnet, daß der zylindrische Steuerkörper (20) eine von außerhalb des Gehäuses (2) mit im wesentlichen zylindrischem Innenraum (4) betätigbare Handhabe (72) zumindest zur Verstellung in die Schnellablaßstellung aufweist.

10. Ablaßventil nach einem der vorhergehenden Ansprüche, dadurch gekennzeichnet, daß das Ablaßventil mit Schnellablaßfunktion eine Einrichtung (82) zum Normalablaß aufweist.

11. Ablaßventil nach Anspruch 10, dadurch gekennzeichnet, daß die Normalablaßeinrichtung eine Kammer, deren Innenraum (4) mit dem Anschluß (12, 13, 7) zur Manschette (9) verbunden ist und ein zumindest teilweise in der Kammer befindliches Hohlteil (82) mit elastischen Wandungen aufweist, wobei in mindestens einer der in der Kammer befindlichen Wandungen ein Schlitz (124) vorgesehen ist und die Kammer im übrigen dichtend geschlossen ist und wobei der Innenraum des Hohlteils mit der Atmosphäre verbunden ist, und die Öffnung des Schlitzes (124) beeinflussendes Element (70) aufweist und das Element von außen steuerbar verstellbar ausgebildet ist.

12. Ablaßventil nach Anspruch 11, dadurch gekennzeichnet, daß das Hohlteil (82) mit elastischen Wandungen im Inneren des zylindrischen Steuerkörpers (20) angeordnet ist und die Schlitze (124) mit einer Öffnung (17) im zylindrischen Körper (20) in Verbindung stehen, welche Öffnung sich in dem Teil des zylindrischen Körpers befindet, der durch die Dichtung (80) gegen die Atmosphäre abgedichtet ist.

13. Ablaßventil nach einem der Ansprüche 3 bis 12, dadurch gekennzeichnet, daß der Anschluß an die Atmosphäre als an der dem Pumpenanschluß (6, 6') abgewandten, offenen Endfläche des Gehäuses (2) angebrachte Kappe (40) ausgebildet ist, durch eine Öffnung (52) derselben sich eine Verlängerung (72) geringeren Durchmessers des zylindrischen Körpers nicht dichend erstreckt, so daß die Öffnung (52) als Luftauslaß, die Kappe als Anschlag für den zylindrischen Körper und die Verlängerung (72) als Handhabe zum Verschieben des zylindrischen Körpers in die Schnellablaßstellung dient.

14. Ablaßventil nach Anspruch 13, dadurch gekennzeichnet, daß die Verlängerung (72) gegenüber dem zylindrischen Körper (20) verstellbar ausgebildet ist und das die Öffnung des Schlitzes (124) beeinflussende Element (76) verstellt.

15. Ablaßventil für ein Blutdruckmeßgerät nach einem der vorgehenden Ansprüche, welches Ablaßventil (1) durch eine Verschiebung eines, im wesentlichen kreiszylindrischen, ersten Verstellelementes (20) in einem Ventilgehäuse (2) mit im wesentlichen kreiszylindrischem Innenraum (4), aus einer ersten, stabilen Stellung (Fig. 2) in eine stabile Schnellablaßstellung (Fig. 3, Fig. 11) umstellbar ist, und bei dem die Meßablaßgeschwindigkeit durch Verdrehen eines zweiten Verstellelementes (70), relativ zum ersten Verstellelement (20) um die Zylinderachse des letzteren, einstellbar ist, dadurch gekennzeichnet, daß an der Innenwand (105) des Innenraums (4) ein, in Umfangsrichtung im wesentlichen unverschiebliches, Führungselement (66) angeordnet ist, das in eine im Außenmantel (24) des kreiszylindrischen ersten Verstellelementes (20) ausgebildete Axialnut (26) eingreift.

16. Ablaßventil nach Anspruch 15, dadurch gekennzeichnet, daß die Axialnut (26) in Richtung (A) der Verschiebung in die Schnellablaßstellung abgeschlossen ist, so daß der Abschluß als Anschlag bei einer Verschiebung in die erste stabile Stellung dient.

17. Ablaßventil nach Anspruch 15 oder 16, dadurch gekennzeichnet, daß das zweite Verstellelement (70) über einen Teil (72) seiner axialen Länge einen Polygonquerschnitt, vorzugsweise hexagonal, aufweist, und daß in der Schnellablaßstellung zumindest ein Teil der axialen Länge mit Polygonquerschnitt im Bereich des Führungselementes (62) ist, wobei die Umfangslinie des Polygonquerschnitts so bemessen ist, daß das Führungselement (62) eine Drehung des zweiten Verstellelements (70) relativ zum Ventilgehäuse (2) nur um einen kleinen Winkel erlaubt, während in der ersten stabilen Stellung der Teil der axialen Länge mit Polygonquerschnitt außerhalb des Bereichs des Führungselementes liegt.

18. Ablaßventil nach einem der Ansprüche 15 bis 17, dadurch gekennzeichnet, daß das Führungselement als U-förmig gebogenes Drahtstück (62) ausgebildet ist, das am Ventilgehäuse (2) derart angebracht ist, daß sein einer Schenkel (66) in axialer Richtung (A) entlang der Innenwand des zylindrischen Innenraumes (4) in diesen (4) hineinragt, daß die U-förmige Umbiegung (68) die Umfangswand (14) des zylindrischen Innenraums zumindest teilweise überspannt, und daß sein anderer Schenkel (64), in der Umfangswand, vorzugsweise in einer axialen Bohrung (18), oder an der Außenseite der Umfangswand, gegen eine Verschiebung in Umfangsrichtung festgelegt ist.

19. Ablaßventil nach einem der Ansprüche 15 bis 18 dadurch gekennzeichnet, daß eine, den zylindrischen Innenraum in Richtung der ersten stabilen Stellung abschließende, Kappe (40) auf dem Ventilgehäuse (2) drehbar angebracht ist, durch eine Mittenöffnung (52) derselben das zweite Verstellelement axial verschieblich hindurchragt, die Mittenöffnung (52) derart an die Querschnittsform (72) des zweiten Verstellelementes (70) angepaßt ist, daß das zweite Verstellelement drehfest mit der Kappe (40) verbunden ist, und daß die Kappe axial unverschieblich angeordnet ist.

20. Ablaßventil nach Anspruch 19, dadurch gekennzeichnet, daß eine Winkelbegrenzung (58, 60; 64) für die Verdrehung der Kappe vorgesehen ist.

21. Ablaßventil nach Anspruch 19 oder 20, dadurch gekennzeichnet, daß in der Seitenwand (42) der Kappe (40) eine Umfangsnut (44) ausgebildet ist, die teilweise zu mindestens einems ins Innere (50) der Kappe offenen Schlitz (46, 48) ausgebildet ist, daß in der Umfangswandung (14) des zylindrischen Innenraums (4) unter der Kappe eine entsprechende Nut (16) ausgebildet ist, und daß ein Befestigungselement (54) vorhanden ist, das in die Nut (44) der Kappe, und durch den Schlitz (46, 48) in die Nut (16) in der Umfangswandung (14) eingreift.

22. Ablaßventil nach Anspruch 21, dadurch gekennzeichnet, daß das Befestigungselement als, im wesentlichen ringförmiger, Draht (54) ausgebildet ist, von dem ein Teil (56; 58, 60) seiner Länge so zur Mitte des Rings 54 aus- oder umgebogen ist, daß er durch den Schlitz (46; 48) in die Wandungsnut (16) eingreift.

23. Ablaßventil nach Anspruch 21 oder 22, dadurch gekennzeichnet, daß in der Wandungsnut (16) ein Anschlag (64) für das in sie (16) eingreifende Befestigungselement (54; 58, 60) angeordnet ist.

24. Ablaßventil nach Anspruch 18 und 23, dadurch gekennzeichnet, daß das U-förmig gebogene Drahtstück (62) die Wandungsnut (16) in Axialrichtung (A) durchdringt und, mit seinem Schenkel (64), als Anschlag dient.

25. Ablaßventil nach einem der Ansprüche 21 bis 24, dadurch gekennzeichnet, daß der Schlitz bzw. die Schlitze (46, 48) in der Seitenwand (42) der Kappe (40) eine in Umfangsrichtung größere Ausdehnung als die ihn bzw. sie durchgreifenden Teile (56; 58, 60) des Befestigungselementes (54) aufweist, bzw. aufweisen, und daß diese Teile, im Falle mehrere Schlitze, relativ zu den Schlitzen und zueinander so angeordnet sind, daß die Kappe relativ zum Befestigungselement verdrehbar ist.

**Revendications**

1. Soupape de décharge présentant une fonction de décharge rapide, destinée à un sphygmomanomètre, comprenant une pompe (5) pour gonfler un brassard (9), soupape qui peut passer d'une première position stable (figure 2) à une position stable de décharge rapid (figure 3, figure 11), caractérisée en ce que la soupape d'évacuation (1) est amenée à la première position stable en actionnant la pompe de gonflage.

2. Soupapge de décharge selon la revendication 1, caractérisé en ce que la soupape de décharge (1) est amenée à la première position par la pression ou par l'aspiration de la pompe (5).

3. Soupape de décharge selon la revendication 2, caractérisée en ce que la soupape de décharge (1) comprend un boîtier de soupape (2) comportant une enceinte interne (4) sensiblement cylindrique, à laquelle sont raccordées (6', 13/52) la connexion (6) avec la pompe, la connexion (12) avec le brassard (9) et l'atmosphère, et en ce que dans l'enceinte interne cylindrique est disposé de façon mobile un élément de commande (20) sensiblement cylindrique qui réalise ou autorise dans une première position (figure 2) la liaison entre le raccord de la pompe et le raccord du brassard tout en réalisant l'étanchéité vis-à-vis de l'atmosphère, et dans une seconde position (figure 3, figure 11) (position de décharge rapide) réalise ou autorise la liaison entre le raccord du brassard et l'atmosphère tout en réalisant l'étanchéité vis-à-vis du raccord de la pompe, de manière qu'une presson de la pompe (ou éventuellement une aspiration de la pompe) fasse passer l'élément de commande cylindrique à la première position (figure 2).

4. Soupape de décharge selon la revendication 3, caractérisée en ce que l'ouverture de passage (6) allant vers la pompe et l'ouverture de passe (12) allant vers le brassard sont séparées d'une certain distance en direction axiale de l'enceinte interne (4), en ce que l'élément de commande (20) peut se déplacer dans l'enceinte interne du boîtier de la soupape dans sa direction axiale et subdivise l'enceinte interne en deux chambres partielles d'importance variable et étanches l'une par rapport à l'autre, et en ce que l'élément de commande peut être déplacé dans deux positions, les deux ouvertures de passage (6, 12) débouchant, dans la première position (figure 2), dans la première chambre interne (11) qui est fermée pour le reste, et l'ouverture de passage (6) allant à la pompe (5) débouche, dans la seconde position (figure 3), dans la première chambre interne alors que l'ouverture de passage (12) allant au brassard (9) débouche dans la seconde chambre interne qui est ouverte à l'atmosphère (132, 52) au moins dans la seconde position de déplacement de l'élément de commande.

5. Soupape de décharge selon la revendication 3 ou 4, caractérisée en ce que le raccord (6, 6') de la pompe (5), le raccord (12, 13) du brassard et le raccord (132, 52) avec l'atmosphère sont raccordés selon cette séquence, vus en direction axiale, à l'enceinte interne du boîtier (2) de la soupape, et l'élément de commande cylindrique (20) est relié de façon étanche (80) et de façon glissante à la paroi interne du cylindre creux sur au moins une partie de sa longueur, qui n'est cependant pas supérieure à la distance séparant les bords tournés l'une vers l'autre des raccords, la région rendue étanche (80) se trouvant dans la première position entre le raccord du brassard et l'atmosphère et dans la position de décharge rapide entre le raccord avec le brassard et le raccord de pression (6) avec la pompe.

6. Soupape de décharge selon la revendication 5, caractérisée en ce que la région d'étanchéité est constituée sous forme d'un joint torique (80) maintenu dans une gorge périphérique (22) de l'élément de commande cylindrique (20).

7. Soupape de décharge selon la revendication 5 ou 6, caractérisée en ce que l'élément de commande (20) est maintenu dans la position qu'il occupe par frottement de l'élément d'étanchéité (80).

8. Soupape de décharge selon l'une quelconque des revendications précédentes, caractérisée en ce que la soupape de décharge peut être amenée manuellement à la position de décharge rapide et stable.

9. Soupape de décharge selon l'une quelconque des revendications 3 à 8, caractérisée en ce que l'élément de commande cylindrique (20) comprend und poignée (72) pouvant être manoeuvrée de l'extérieur du boîtier (2) et dans l'enceinte interne (4) sensiblement cylindrique, au moins pour passer à la position de décharge rapide.

10. Soupape de décharge selon l'une quelconque des revendications précédentes, caractérisée en ce que la soupape de décharge à fonction de décharge rapide comprend un dispositif (82) pour une décharge normale.

11. Soupape de décharge selon la revendication 10, caractérisée en ce que le dispositif de décharge normale comprend une chambre dont l'enceinte interne (4) est reliée au raccord (12, 13, 5) du brassard (9) et une partie creuse (82) à parois élastiques se trouvant au moins partiellement dans la chambre, une fente (124) étant prévue dans au moins l'une des parois se trouvant dans la chambre et la chambre étant, pour le reste, fermée de façon étanche, l'enceinte interne de la partie creuse étant reliée à l'atmosphère et comprenant un élément (70) ayant une influence sur l'ouverture de la fente (124), élément constitué pour pouvoir être déplacé de façon commandable depuis l'extérieure.

12. Soupape de décharge selon la revendication 11, caractérisée en ce que la partie creuse (82) à parois élastiques est disposée à l'intérieure de l'élément de commande cylindrique (20) et les fentes (124) sont en liaison avec une ouverture (17) de l'élément cylindrique (20), laquelle ouverture se trouve dans la partie de l'élément cylindrique qui est rendue étanche vis-à-vis de l'atmosphère par la garniture (80).

13. Soupape de décharge selon l'une quelconque des revendications 3 à 12, caractérisée en ce que le raccord avec l'atmosphère est constitué sous forme d'un couvercle (40) monté sur la surface d'extrémité du boîtier (2) qui est ouverte et à l'opposé du raccord (6, 6') de la pompe, un prolongement (72) de plus petit diamètre de l'élément cylindrique traversant de façon non étanche une ouverture (52) dudit couvercle, ce qui fait que l'ouverture (52) sert de décharge pour l'air, le couvercle sert de butée pour l'élément cylindrique et le prolongement (72) sert de poignée pour déplacer l'élément cylindrique vers la position de décharge rapide.

14. Soupape de décharge selon la revendication 13, caractérisée en ce que le prolongement (72) est prévu pour être mobile par rapport à l'élément cylindrique (20) et déplace l'élément (76) qui a une influence sur la fente (124).

15. Soupape de décharge pour sphygmomanomètre selon l'une quelconque des revendications précédentes, cette soupape de décharge (1) pouvant être amenée d'une première position stable (figure 2) à une position de décharge rapide stable (figure 3, figure 11) par le déplacement d'un premier élément de réglage (20) de forme sensiblement cylindrique et circulaire dans un boîtier (2) de la soupape à enceinte interne sensiblement cylindrique et circulaire (4), et dans laquelle la vitesse de décharge de mesure peut être réglée en faisant tourner un second élément de réglage (70) par rapport au premier élément de réglage (20) autour de l'axe cylindrique de ce dernier, caractérisée en ce qu'un élément de guidage (66) qui est sensiblement bloqué en direction périphérique est disposé contre la paroi interne (105) de l'enceinte interne (4), élément qui pénètre dans une gorge axiale (26) constituée dans l'enveloppe extérieure (24) du premier élément de réglage circulaire et cylindrique (20).

16. Soupape de décharge selon la revendication 15, caractérisée en ce que la gorge axiale (26) est fermée en direction (A) du déplacement vers la position de décharge rapide, de manière que la fermeture serve de butée lors du déplacement vers la première position stable.

17. Soupape de décharge selon la revendication 15 ou 16, caractérisée en ce que le second élément de réglage (70) présente sur une partie (72) de sa longueur axiale une section transversale polygonale et de préférence hexagonale, et en ce que dans la position de décharge rapide, une partie au moins de la longueur axiale de section polygonale se trouve dans la région de l'élément de guidage (62), la ligne périphérique de la section polygonale étant dimensionnée de manière que l'élément de guidage (62) autorise une rotation du second élément de réglage (70) par rapport au boîtier (2) de la soupape seulement sur un angle limité, alors que dans la première position stable, la partie de la longueur axiale de section polygonale se trouve située à l'extérieur de la région de l'élément de guidage.

18. Soupape de décharge selon l'une quelconque des revendications 5 à 17, caractérisée en ce que l'élément de guidage est constitué sous forme d'un fil replié en U (62) qui est monté dans le boîtier (2) de la soupape de manière que l'une de ses branches (66) pénètre en direction axiale (A) le long de la paroi interne de l'enceinte interne cylindrique (4) dans cette dernière, en ce que la partie courbe en forme de U (68) recouvre au moins en partie la paroi périphérique (14) de l'enceinte interne cylindrique, et en ce que son autre branche (64) est bloquée de manière à ne pouvoir se déplacer en direction périphérique dans la paroi périphérique, de préférence dans un alésage axial (18), ou sur le côté extérieur de la paroi périphérique.

19. Soupape de décharge selon l'une quelconque des revendications 15 à 18, caractérisée en ce qu'un couvercle (40) fermant l'enceinte interne cylindrique en direction de la première position stable est monté de façon rotative sur le boîtier (2) de la soupape, traverse de façon mobile et axialement le second élément de réglage en passant par une ouverture centrale (52) du boîtier, en ce que l'ouverture centrale (52) est adaptée à la forme en section transversale (72) du second élément de réglage (70) de manière que le second élément de réglage soit solidaire en rotation avec le couvercle (40), et en ce que le couvercle est monté de façon à ne pas pouvoir se déplacer axialement.

20. Soupape de décharge selon la revendication 19, caractérisée en ce que l'on prévoit une limite angulaire (58, 60; 64) pour la rotation du couvercle.

21. Soupape de décharge selon la revendication 19 ou 20, caractérisée en ce qu'une rainure périphérique (44) est constituée dans la paroi frontale (42) du couvercle et formée en partie jusqu'à au moins une fente (46, 48) ouverte vers l'intérieure (50) du couvercle, en ce que dans la paroi périphérique (14) de l'enceinte interne cylindrique (4) située sur le couvercle est constituée une rainure correspondante (16), et en ce qu'il est prévu un élément de fixation (54) qui pénètre dans la rainure (44) du courvercle et dans la rainure (16) de la paroi périphérique (14) en passant par la fente (46, 48).

22. Soupape de décharge selon la revendication 21, caractérisée en ce que l'élément de fixation est constitué sous forme d'un fil (54) de forme sensiblement circulaire, dont une partie (56; 58, 60) de la longueur est repliée ou recourbée en direction du centre de l'anneau (54) de manière à pénétrer dans la rainure (16) de la paroi en passant par la fente (46; 48).

23. Soupape de décharge selon la revendication 21 ou 22, caractérisée en ce qu'une butée (64) destinée à l'élément de fixation (54; 58, 60) et pénétrant dans celui-ci est disposée dans la rainure (16) de la paroi.

24. Soupape de décharge selon la revendication 18 et 23, caractérisée en ce que le fil replié en forme de U (62) traverse la rainure (16) de la paroi en direction axiale (A) et sert de butée par sa branche (64).

25. Soupape de décharge selon l'une quelconque des revendications 21 à 24, caractérisé en ce que la ou les fentes (46, 48) de la paroi frontale (42) du couvercle (40) a ou ont une dimension plus importante en direction périphérique que les parties (56; 58, 60) de l'élément de fixation qui la ou les traversent, et en ce que ces parties, dans le cas où il existe plusieurs fentes, sont disposées par rapport aux fentres et par rapport à elles-mêmes de manière que le couvercle puisse être tourné par rapport à l'élément de fixation.

**Claims**

1. An outlet valve with a rapid deflation function, for a sphygmomanometer with a pump (5) for inflating a sleeve (9), which valve can be transferred out of a first stable position (Figure 2) into a stable rapid deflation position (Figure 3, Figure 11), characterised in that the outlet valve (1) is transferred into the first stable position by the inflating operation of the pump.

2. An outlet valve as claimed in Claim 1, characterised in that the outlet valve (1) is transferred into the first position by the pressure or suction of the pump (5).

3. An outlet valve as claimed in Claim 1 or 2, characterised in that the outlet valve (1) comprises a valve housing (2) with a substantially cylindrical interior (4) to which the connection (6) to the pump (5), the connection (12) to the sleeve (9) and the atmosphere are connected (6', 13/52), and that a corresponding, substantially cylindrical control member (20) is adjustably mounted in the cylindrical interior, which control member establishes or permits, in a first position (Figure 2), the communication between pump connection and sleeve connection with sealing off from the atmosphere and, in a second position (Figure 3, Figure 11) (rapid deflation position), the communication between sleeve connection and atmosphere with sealing off from the pump connection in such a manner that a pump pressure (or possibly pump suction) sets the cylindrical control member in the first position (Figure 2).

4. An outlet valve as claimed in Claim 3, characterised in that the passage (6) to the pump and the passage (12) to the sleeve are arranged spaced apart in the axial direction of the interior (4), that the control member (20) is displaceable in the interior of the valve housing (2), in the axial direction thereof, and divides the interior into two subspaces of variable size, sealed off from one another, and that the control member can be displaced into two positions in the first of which (Figure 2) both passages (6, 12) open into the first subspace (11), which is otherwise closed, and in the second position (Figure 3), the passage (6) to the pump (5) opens into the first subspace and the passage (12) to the sleeve (9) opens into the second subspace which is open to the atmosphere at least in the second displacement position of the control member (132, 52).

5. An outlet valve as claimed in Claim 3 or 4, characterised in that the connection (6, 6') to the pump (5), the connection (12, 13) to the sleeve and the connection (132, 52) to the atmosphere are connected to the interior of the valve housing (2) in this sequence, seen in the axial direction, and the cylindrical control member (20) is connected, in a sealing (80) manner and slidably, to the inner wall of the hollow cylinder at least over a portion of its length which is not, however, greater than the spacing between the adjacent edges of the connections, and the sealing region (80) is disposed, in the first position, between connection to the sleeve and to the atmosphere and in the rapid deflation position between the connection to the sleeve and the pressure connection (6) to the pump.

6. An outlet valve as claimed in Claim 5, characterised in that the sealing region is constructed in the form of an O-ring (80) which is held in a circumferential groove (22) in the cylindrical control member (20).

7. An outlet valve as claimed in Claim 5 or 6, characterised in that the control member (20) is held in its particular position by frictional locking of the sealing element (80).

8. An outlet valve as claimed in one of the preceding claims, characterised in that the outlet valve can be transferred manually into the stable rapid deflation position.

9. An outlet valve as claimed in one of the Claims 3 to 8, characterised in that the cylindrical control member (20) comprises a handle (72), which can be actuated from outside the housing (2) with a substantially cylindrical interior (4), at least for setting into the rapid deflation position.

10. An outlet valve as claimed in one of the preceding claims, characterised in that the outlet valve with a rapid deflation function comprises a device (82) for the normal deflation.

11. An outlet valve as claimed in Claim 10, characterised in that the normal deflation device comprises a chamber, the interior (4) of which is in communication with the connection (12, 13, 7) to the sleeve (9) and a hollow member (82) with resilient walls which is at least partially in the chamber, a slot (124) being provided in at least one of the walls which is in the chamber and the chamber being otherwise closed with a sealing effect, and the interior of the hollow member is in communication with the atmosphere and comprises an element (70) influencing the opening of the slot (124) and the element is adapted for adjustment, being controllable from the outside.

12. An outlet valve as claimed in Claim 11, characterised in that the hollow member (82) with resilient walls is disposed in the interior of the cylindrical control member (20) and the slots (124) are in communication with an opening (17) in the cylindrical member (20), which opening is in the portion of the cylindrical member which is sealed off from the atmosphere by the seal (80).

13. An outlet valve as claimed in one of the Claims 3 to 12, characterised in that the connection to the atmosphere is constructed in the form of a cap (40) which is fitted to the open end face of the housing (2) remote from the pump connection (6, 6'), through an opening (52) in which cap an extension (72) of smaller diameter of the cylindrical member extends in a non-sealing manner, so that the opening (52) serves as an air outlet, the cap as a stop for the cylindrical member and the extension (72) as a handle for the displacement of the cylindrical member into the rapid deflation position.

14. An outlet valve as claimed in Claim 13, characterised in that the extension (72) is adapted for adjustment in relation to the cylindrical member (20) and adjusts the element (76) influencing the opening of the slot (124).

15. An outlet valve for a sphygmomanometer as claimed in one of the preceding Claims, which outlet valve (1) can be changed over, by displacement of a substantially circular cylindrical, first adjusting element (20) in a valve housing (2) with a substantially circular cylindrical interior (4), from a first, stable position (Figure 2) into a stable rapid deflation position (Figure 3, Figure 11), and wherein the measuring deflation rate is adjustable by turning a second adjusting element (7) relatively to the first adjusting element (20) about the cylinder axis of the latter, characterised in that disposed on the inner wall (105) of the interior (4) is a guide element (66) which is substantially non-displaceable in the circumferential direction and which engages in an axial groove (26) formed in the outer wall (24) of the circular cylindrical first adjusting element (20).

16. An outlet valve as claimed in Claim 15, characterised in that the axial groove (26) is closed in the direction (A) of displacement into the rapid deflation position so that the closure serves as a stop on displacement into the first stable position.

17. An outlet valve as claimed in Claim 15 or 16, characterised in that the second adjusting element (70) has a polygonal cross-section, preferably hexagonal, over a portion (72) of its axial length and that in the rapid deflation position at least a portion of the axial length with a polygonal cross-section is in the region of the guide element (62), the circumferential line of the polygonal cross-section being so dimensioned that the guide element (62) only permits a rotation of the second adjusting element (70) through a small angle in relation to the valve housing (2), while in the first stable position the portion of the axial length with a polygonal cross-section lies outside the range of the guide element.

18. An outlet valve as claimed in one of the Claims 15 to 17, characterised in that the guide element is constructed in the form of a piece of wire bent into U-shape which is fitted to the valve housing (2) in such a manner that its one arm (66) projects, in the axial direction (A), along the inner wall of the cylindrical interior (4), into this (4), that the U-shaped bend (68) at least partially straddles the circumferential wall (14) of the cylindrical interior and that its other arm (64) is secured against displacement in the circumferential direction in the circumferential wall, preferably in an axial bore (18), or at the outside of the circumferential wall.

19. An outlet valve as claimed in one of the Claims 15 to 18, characterised in that a cap (40), which closes the cylindrical interior in the direction of the first stable position, is mounted for rotation on the valve housing (2) and through a central opening (52) therein, the second adjusting element projects for axial displacement, the central opening (52) being adapted to the cross-sectional shape (72) of the second adjustment element (70) in such a manner that the second adjusting element is connected to the cap (40) for rotation therewith but not in relation thereto and that the cap is held against axial displacement.

20. An outlet valve as claimed in Claim 19, characterised in that an angular limit stop (58, 60; 64) is provided for the rotation of the cap.

15

21. An outlet valve as claimed in Claim 19 or 20, characterised in that formed in the side wall (42) of the cap (40) is a circumferential groove (44) which is at least partially constructed in the form of a slot (46, 48) open in the interior (50) of the cap, that a corresponding groove (16) is formed in the circumferential wall (14) of the cylindrical interior (4), under the cap, and that a securing element (54) is present which engages in the groove (44) of the cap and through the slot (46, 48) into the groove (16) in the circumferential wall (14).

22. An outlet valve as claimed in Claim 21, characterised in that the securing element is constructed in the form of a substantially annular wire (54), a portion (56; 58, 60) of the length of which is bent in or round towards the middle of the ring 54 so that it engages through the slot (46; 48) into the groove (16) in the wall.

23. An outlet valve as claimed in Claim 21 or 22, characterised in that a stop (64) for the securing element (54; 58, 60) engaging in groove (16) in the wall is provided in the groove (16).

24. An outlet valve as claimed in Claims 18 and 23, characterised in that the piece of wire (62) which is bent into U-shape penetrates through the groove (16) in the wall in the axial direction (A) and serves as a stop with its arm (64).

25. An outlet valve as claimed in one of the Claims 21 to 24, characterised in that the slot or slots (46, 48) in the side wall (42) of the cap (40) has or have a greater extent in the circumferential direction than the parts (56; 58, 60) of the securing element (54) engaging through it or them and that these parts, in the case of a plurality of slots, are arranged in relation to the lots and to one another so that the cap is rotatable in relation to the securing element.

Fig. 1

Fig. 2

0 079 617

Fig. 3

Fig.4

Fig.5

*Fig.6A*

*Fig.6B*

Fig. 7

56
15
14
16    16    15
58
4

Draht    0,6 φ

Fig.8

54

56    58~
60~

68
Fig.9
62 →
66    64

Fig.10A

70

78

Fig.10B

70

72

74

74

76

78

Fig.11